(19) Europäisches Patentamt / European Patent Office / Office européen des brevets

(11) EP 4 431 512 A1

(12) EUROPEAN PATENT APPLICATION

(43) Date of publication: **18.09.2024 Bulletin 2024/38**

(21) Application number: **23162389.3**

(22) Date of filing: **16.03.2023**

(51) International Patent Classification (IPC): ***C07D 498/04*** *(2006.01)* ***A61P 5/24*** *(2006.01)* ***A61K 31/5383*** *(2006.01)*

(52) Cooperative Patent Classification (CPC): **C07D 498/04; A61P 5/24**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Bayer Consumer Care AG**
**4052 Basel (CH)**

(72) Inventors:
- **ROTTMANN, Antje**
  **13089 Berlin (DE)**
- **PETERS, Michaele**
  **10405 Berlin (DE)**
- **ENGELEN, Anna**
  **45149 Essen (DE)**
- **BORDINI, Elenia**
  **37135 Verona (IT)**
- **DAVALLI, Silvia**
  **37135 Verona (IT)**
- **CUFARI, Domenico**
  **37135 Verona (IT)**
- **TROWER, Mike**
  **Stevenage Hertfordshire SG1 2FX (GB)**

(74) Representative: **BIP Patents**
**c/o Bayer Intellectual Property GmbH**
**Alfred-Nobel-Straße 50**
**40789 Monheim am Rhein (DE)**

# (54) NOVEL DUAL NK-1/NK-3 RECEPTOR ANTAGONISTS

(57) The present invention relates 2-(3,5-dimethylphenyl)-N-[4-(4-fluorophenyl)-3-pyridyl]-N,2-dimethyl-propanamide compounds of general formula (I), methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of sex-hormone dependent diseases, as a sole agent or in combination with other active ingredients.

EP 4 431 512 A1

## Description

The present invention covers 2-(3,5-dimethylphenyl)-N-[4-(4-fluorophenyl)-3-pyridyl]-N,2-dimethyl-propanamide compounds of general formula (I) as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of sex-hormone dependent diseases, as a sole agent or in combination with other active ingredients.

## FIELD OF THE INVENTION

This invention relates to a method of treatment of sex-hormone dependent diseases, in a mammal, for which either gonadotropin suppression and/or androgen suppression and/or suppression of KNDy neuron activation caused by estrogen, testosterone withdrawl due to menopause or antihormonal cancer therapy is desired. Gonadotropin and/or androgen suppression and/or suppression of KNDy neuron activation is achieved according to this invention by administration of a dual neurokinin-1 (NK-1) receptor antagonist and neurokinin-3 (NK-3) receptor antagonist (herein called dual NK-1/NK-3 receptor antagonists). Suitable dual NK-1/NK-3 receptor antagonists useful in this invention are represented by the compounds described herein.

## BACKGROUND OF THE INVENTION

It has been found that KNDy neuron suppression by dual NK-1/NK-3 receptor antagonists results in beneficial effects, in a number of sex hormone-dependent diseases and clinical applications, in males and in females, such as vasomotor symptoms (hot flashes), insomnia, sleep distubrances, night-time awakenings and other hormone dependet diseases.

Recent data have shown that a group of sex steroid-responsive neurons in the hypothalamic infundibular (arcuate) nucleus coexpress kisspeptin, dynorphin as well as neurokinin B (NKB); the so called kisspeptin/neurokinin B/dynorphin neurons or KNDy neurons (Lehman MN et al, Endocrinology (2010);151(8):3479-89). Morphologic studies have shown that KNDy neurons from postmenopausal women are hypertrophied (Rance NE, et al, J Clin Endocrinol Metab. (1990);71(1):79-85) and this hypertrophy is accompanied by elevated NKB, kisspeptin and substance P (SP) gene expression (Race NE (1990); Rance NE and Young WS, Endocrinology (1991);128(5):2239-47; and Rometo AM and Rance NE, J Neuroendocrinol. (2008);20(12):1376-81).

These expression changes in women are a consequence of estrogen withdrawal (Abel TW et al, J Clin Endocrinol Metab. (1999);84(6):2111-8; Sandoval-Guzmán T, et al, J Neuroendocrinol. (2004);16(2):146-53; and Rometo AM, et al, J Clin Endocrinol Metab. (2007);92(7):2744-50). In men these consequences are caused by testerone withdrawal. Estrogen or testosterone sensitive KNDy neurons in the hypothalamus of women have been identified as the control center for thermoregulation that is responsive to both estrogen and ambient temperature (Rance NE et al, Front Neuroendocrinol. (2013);34(3):211-27). In the menopausal state the KNDy neurons are in a state of hyperactivation which disrupts baseline thermoregulation and triggers hot flashes (Rance NE (2013); and Knobf MT, Oncologist. (2006);11(2):96-11). Therefore, modulation of the KNDy neurons control hot flashes in perimenopausal, menopausal and postmenopausal women, as well as in women experiencing estrogen withdrawal, e.g. though anti-cancer therapy with aromatase inhitors or tamoxifen. Likewise, also men undergoing androgen deprivation therapy (ADT) for prostate cancer can experience hot flahes as a common advers effect (Iversen P et al, Prostate Cancer and Prostatic Diseases (2011) 14, 184-190). More recently it has been shown that a dual NK1,3 receptor antagonist shows clinical efficacy for treatment of vasomotor symptoms and associated sleep disturbance (Simon EA et al Menopause 2023 Jan 31. doi: 10.1097/GME.0000000000002138. Online ahead of print).

Further, the gonadotropin-releasing hormone is dysregulated. The gonadotropin-releasing hormone (GnRH), also referred to as luteinizing hormone-releasing hormone (LHRH), is a decapeptide that plays a key role in human reproduction. The hormone is released from the hypothalamus and acts on the pituitary gland to stimulate the biosynthesis and secretion of luteinizing hormone (LH) and follicle stimulating hormone (FSH). LH released from the pituitary gland is primarily responsible for the regulation of gonadal steroid production in both sexes, whereas FSH regulates spermatogenesis in males and follicular development in females. Dual NK1,3 receptor antagonism has been shown to reduce LH secretion and suppress sex hormones in women (Pawsey S et al. JCEM 2021, Vol. 106, No. 8) and NK3 receptor antagomism suppress LH secretion and sex hormones in men and women (Fraser GL et al Endocrinology 2015, 156(11).

It has been found that androgen, estrogen and/or gonadotropin suppression or abrogation results in beneficial effects, in a number of diseases and clinical applications, in males and in females.

In men these diseases or disorders, include, but are not limited to benign prostatic hyperplasia (BPH), metastatic prostatic carcinoma, breast cancer, testicular cancer, androgen dependent acne, hypertrichosis, seborrhoea, male pattern baldness and precocious puberty in boys.

In women, suppression of KNDy neuron activity by dual NK-1/NK-3 receptor antagonists, has been shown to be therapeutic for diseases and/or symptoms associated with menopause (including pre-menopause and post- menopause) in clinical settings, such as, for example, vasomotor symptoms, sleep disturbances and night-time awakenings.

In women, estrogen and/or gonadotropin suppression or abrogation by dual NK-1/NK-3 receptor antagonists, in clinical settings, such as, for example, in treating Polycystic Ovarian Syndrome (PCOS), endometriosis, adenomyosis, uterine fibroids, heavy menstrual bleeding, hirsutism, androgen dependent acne, seborrhoea, female androgenetic alopecia and hypertrichosis, gonadal steroiddependent neoplasm (breast cancer, ovary cancer, etc.), gonadotropin-producing pituitary adenoma, premenstrual syndrome and sterility (e.g. assisted reproductive techniques such as in vitro fertilization). Other related conditions affecting women are pre-eclampsia and pregnancy prevention (contraception), and for both sexes, hidradenitis suppurativa, vasomotor symptoms (hot flushes), sleep disturbances and night-time awakenings.

As is the case in female and male subjects, however, gonadotropin and estrogen or androgen suppression (also called estrogen or androgen deprivation, respectively) is accompanied by a variety of undesirable clinical conditions and symptoms. For males receiving gonadotrophin releasing hormone (GnRH) agonists these initially cause a flare of increased androgen production that in patients with sex-hormone sensitive cancers may cause a short term increase in tumour growth and if the cancer has spread to the bone it may result in pain. These agents are also injected so there could be soreness, swelling and redness at the injection site. Other side effects that are also associated with GnRH antagonists are for example reduced or absent libido, impotence, shrinkage of testicles and penis, hot flushes, breast tenderness or growth of breasts, osteoporosis, anemia, cognitive reduction, muscle mass loss, weight gain, fatigue, elevated cholesterol and depression.

For male subjects administered 5-alpha-reductase inhibitors side effects are sexual in nature for example erectile dysfunction, decreased libido, reduced ejaculate and breast enlargement or tenderness. These drugs can also lower levels of Prostate-Specific Antigen (PSA) thereby interfering with the results of this analyte utilised to detect prostate cancer. Male and female subjects may also experience with spironolactone breast tenderness, menstrual irregularities (females only), fatigue, dizziness, confusion, nausea and vomiting, headache, hypotension, diarrhoea and possibly hyperkalemia. Cyproterone acetate can cause oligomenorrhea (females only), melasma, fluid retention, nausea and vomiting; this drug is also in rarer cases associated with liver hepatotoxicity and clotting disorders. Flutamide administration may lead to breast tenderness, gastrointestinal upset, hot flashes, and decreased libido; it has a serious potential side-effect of hepatotoxicity. Females taking combined oral contraceptives may experience metrorrhagia, nausea, vomiting, breast tenderness and headaches; and more rarely blood clots, myocardial infarctions, stroke, abnormal lipid indices, glucose intolerance and hypertension.

Compounds showing selective affinity simultaneously to both NK-1 and NK-3 receptors namely dual NK-1/NK-3 receptor antagonist elinzanetant have been shown to be effective in and are being developed for the treatment of sex hormone-dependet diseases, such as e.g. vasomotor symptoms, sleep disturbances associated with menopause, and night time awakenings associated with menopause.

There is a need for providing novel and further dual NK-1/NK-3 receptor antagonist show comparable or superior biological activity as does elinzanetant to treat sex hormone-dependent diseases, like vasomotor symptoms, sleep disturbances and/or night time awakenings.

## SUMMARY OF THE INVENTION:

The present invention provides novel dual NK-1/NK-3 receptor antagonists to achieve gonadotropin and/or androgen or estrogen suppression, and/or suppression of KNDy neuron hyperactivation caused by reduction or withdrawl of sex hormone levels, or caused by antagonizing sex hormone activity in mammals. According to this invention, these novel dual NK-1/NK-3 receptor antagonists are usefull for the treatment of diseases which are caused by abnormal levels of sex-hormones, particularly testosterone or estrogen.

Thus, the solution provided by the present invention of the provision of the novel dual NK-1/NK-3 receptor antagonists, in particular dual NK-1/NK-3 receptor antagonists that are oxidized forms of elinzanetant, more particular compounds of general formula (I) as disclosed herein. Hence, the invention relates to oxidized forms of elinzanetant, particularly compounds of general formula (I).

These novel dual NK-1/NK-3 receptor antagonists are particulary usefull in the treatment of sex-hormone dependent diseases.

Hence, in a one aspect, the invention provides a method of treatment of sex-hormone dependent diseases comprising administering to a human in need thereof an effective amount of dual NK-1/NK-3 receptor antagonists according to the invention or a pharmaceutically acceptable salt thereof.

In a further aspect thereof, the invention provides the use of dual NK-1/NK-3 receptor antagonists according to the invention or a pharmaceutically acceptable salt thereof for the manufacture of a medicament for the treatment of

sex-hormone dependent diseases.

In a yet further aspect thereof, the invention provides dual NK-1/NK-3 receptor antagonists according to the invention or a pharmaceutically acceptable salt thereof for use in the treatment of sex-hormone dependent diseases.

In a yet further aspect thereof, the invention provides a method for providing oral contraception, said method comprising administering to the patient an effective amount of dual NK-1/NK-3 receptor antagonists according to the invention.

In a further aspect of the invention there is provided the use of dual NK-1/NK-3 receptor antagonists according to the invention for the production of a drug for oral contraception.

## DETAILED DESCRIPTION OF THE INVENTION

Surprisingly, it has now been found that oxidized forms of elinzanetant are effective in suppressing NK-1 and NK-3 receptor activity. Suppression of the NK-1 and the NK-3 receptor activity has been shown to be beneficial in treatment of certain diseases, in particular treatment of sex-hormone dependent diseases. Hence, the instant invention also relates a method of treatment of sex-hormone dependent diseases which are affected, or exacerbated by, elevated and/ or abnormal levels of gonadotropins and/or estrogens and/ or androgens, the method comprising administering to a subject an effective amount of a dual NK-1/NK-3 receptor antagonist according to the invention. Specifically, the invention relates to a method of suppressing gonadotropin and/or androgen blood levels and/or suppressing KNDy neuron hyperactivity caused by reduction or withdrawl of sex hormone levels or by antagonizing sex hormone activity, by administering an effective amount of a dual NK-1/NK-3 receptor antagonist according to the invention. Further, the invention relates to a method of suppressing pulsatile blood levels of LH by administering an effective amount of a dual NK-1/NK-3 receptor antagonist according to the invention. The compounds defined herein exhibit the ability to suppress NK-1 and NK-3 receptor and thereby gonadotropin and/or androgen or estrogen production, and in particular, are effective in reversibly lowering blood levels and pulses of LH and/or testosterone or estrogen.

In accordance with oneaspect, the present invention covers compounds of general formula (I):

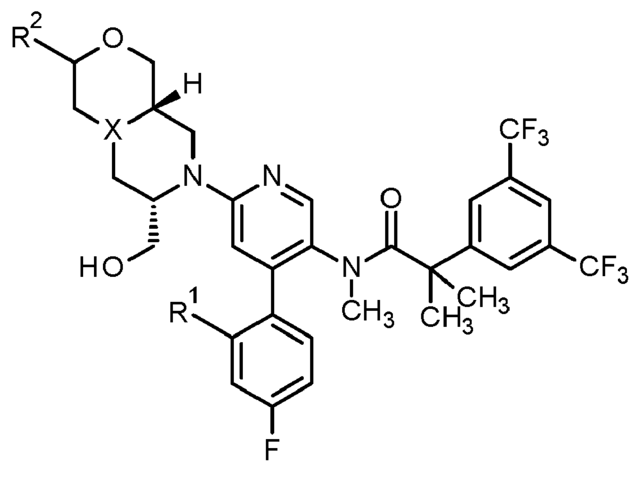

(I);

in which:

$R^1$ is $CH_3$ or $CH_2OH$;

$R^2$ is hydrogen or OH;

X is N or N-oxide.

and hydrates, solvates, and salts thereof, and mixtures of same.

In accordance with an embodiment of the first aspect, the present invention relates to compounds of formula (I) supra, wherein:

(i) $R^1$ is $CH_3$, $R^2$ is OH and X is N, or

(ii) $R^1$ is $CH_2OH$, $R^2$ is hydrogen and X is N, or

(iii) $R^1$ is $CH_2OH$, $R^2$ is OH and X is N, or

(iv) $R^1$ is $CH_3$, $R^2$ is hydrogen and X is N-oxide.

and hydrates, solvates, and salts thereof, and mixtures of same.

One aspect of the invention are compounds of formula (I) as described in the examples, as characterized by their names and/or their structures as well as the subcombinations of all residues specifically disclosed in the compounds of the examples.

A further aspect of the invention are compounds of formula (I), which are present as their salts.

It is to be understood that the present invention relates to any sub-combination within any embodiment or aspect of the present invention of compounds of general formula (I), supra. More particularly still, the present invention covers compounds of general formula (I) which are disclosed in the Example section of this text, *infra.*

The compound according ot the present invention is preferably selected from the group consisting of

N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide;
2-[3,5-bis(trifluoromethyl)phenyl]-N-{4-[4-fluoro-2-(hydroxymethyl)phenyl]-6-[(7S,9aS)-7-(hydroxymethyl)-3-methylhexahydropyrazino[2,1-c][1,4]oxazin-8(1H)-yl]pyridin-3-yl}-N,2-dimethylpropanamide;
2-[3,5-bis(trifluoromethyl)phenyl]-N-{4-(4-fluoro-2-methylphenyl)-6-[(7S,9aS)-7-(hydroxymethyl)-3-methylhexahydropyrazino[2,1-c][1,4]oxazin-8(1H)-yl]pyridin-3-yl}-N,2-dimethylpropanamide; and
2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide.

In one embodiment the compound according to the invention is the compound of formula II:

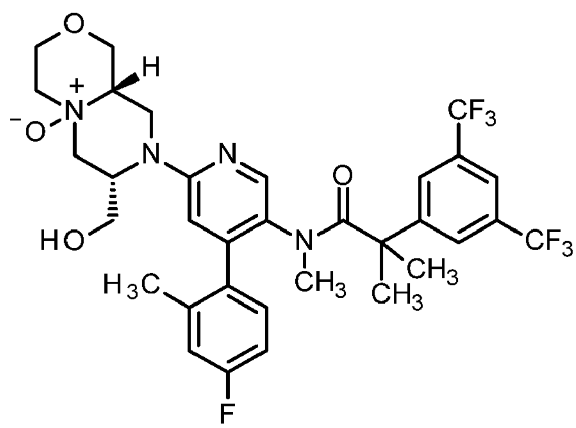

(II).

In one embodiment the compound according to the invention is the compound of formula III:

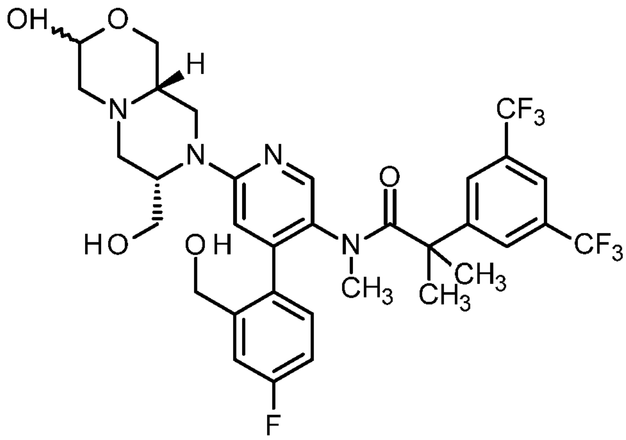

(III).

In one embodiment the compound according to the invention is the compound of formula IV:

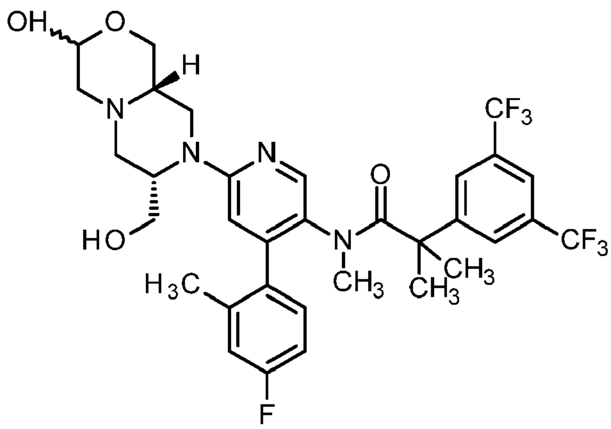

(IV).

In one embodiment the compound according to the invention is the compound of formula V:

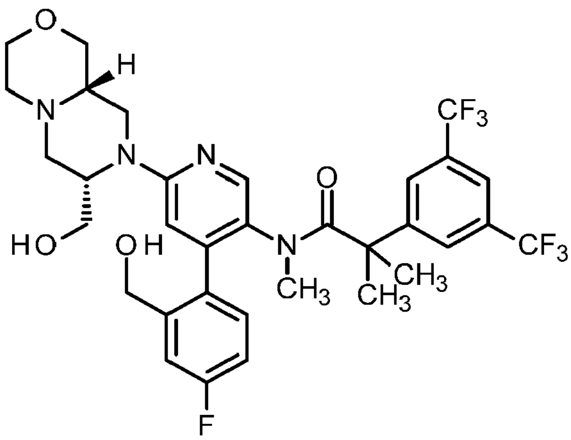

(V).

In accordance with another aspect, the present invention covers methods of preparing compounds of the present invention, said methods comprising the steps as described in the Experimental Section herein.

In other embodiments of the invention, compounds are defined according to the claims as disclosed in the Claims section wherein the definitions are limited according to the preferred or more preferred definitions as disclosed below or specifically disclosed residues of the exemplified compounds and subcombinations thereof.

In certain embodiments, compounds of the present invention have surprising and advantageous properties.

In particular, compounds of the present invention have surprisingly been found to effectively binding NK-1 and NK-3 receptors and antagonizing their activity.

The present invention also relates to useful forms of the compounds as disclosed herein, such as hydrates, solvates, prodrugs, salts, in particular pharmaceutically acceptable salts, and co-precipitates.

The compounds of the present invention can exist as a hydrate, or as a solvate, wherein the compounds of the present invention contain polar solvents, preferably water, methanol or ethanol for example as structural element of the crystal lattice of the compounds. The amount of polar solvents, in particular water, may exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, e.g. as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, paratoluenesulfonic, methansulfonic, 2-naphthalenesulfonic, naphthalenedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and stearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention can be prepared by reacting the compounds of the invention with the appropriate base via any of a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x $CF_3COOH$", "x $Na^+$", for example, are to be understood as not a stoichiometric specification, but simply as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

## Medical Uses

The compounds defined herein exhibit the ability to suppress NK1 and NK3 receptor activity by antagonizing them. Accordingly, they are useful to counteract hyperactivation of KNDy neurons caused by changes in sex hormone levels in the human body. Thereby, they are able to regulate gonadotropin, estrogen and/or androgen production, and in particular, are effective in reversibly dropping the effects of lowering blood levels of LH and the androgen testosterone and/or estrogens like estradiol. Hence, these compounds of the present invention are suited for the treatment of sex hormone-dependent diseases.

The invention thus relates to a method of treating a sex hormone-dependent disease comprising administering to a subject in need thereof a pharmaceutically effective amount of a compound according to the present invention or a pharmaceutically acceptable salt thereof. Likewise the invention relates to a compound according to the present invention, or a pharmaceutically acceptable salt thereof, for use in the treatment of a sex hormone-dependent disease.

The term "sex hormone-dependent disease" as used herein refers to a disease which is exacerbated by, or caused by, excessive, inappropriate including lack of, imbalanced, or unregulated sex hormone production.

In women, sex-hormone dependent diseases or disorders are often associated with the menopause and/or are symptoms of the same. The menopause is characterized by decreased estradiol levels due to ovarian failure, and a resultant increase in gonadotropin releasing hormone (GnRH) secretion from the hypothalamus leading to high gonadotropin (luteinizing hormone (LH) and follicle stimulating hormone (FSH)) concentrations. This dysregulation of the so called HPG-axis through decreased levels of estradiol causes inter alia Vasomotor Symptoms (VMS) in the peri- or post-, and menopausal women.

Accordingly, the invention in particular relates to the compounds according to the invention or a pharmaceutical acceptable salt thereof for use in the treatment or prevention of symptoms of the perimenopause, the menopause, or the postmenopause, preferably selected from a pathological gain of excess body fat and/or excess body weight, insomnia, sleep disturbances and night-time awakenings, anxiety and depression, urinary symptoms of urgency, vasomotor symptoms (VMS) and dysuria. In a particular preferred embodiment the invention relates to a compound according to the invention or a pharmaceutically acceptable salt for the use in the treatment of VMS associated with menopause. Preferably the treatment is the treatment of VMS associated with the perimenopoause, the menopause and/or the post menopause. In a particular preferred embodiment the invention relates to a compound according to the invention or a pharmaceutically acceptable salt for the use in the treatment of insomnia, sleep disturbances or nighttime awakenings, preferably associated with the perimenopoause, the menopause and/or the post menopause.

In some aspects, the present invention provides comopunds thereof for use in the treatment or prevention of the symptoms associated with the andropause selected from a pathological gain of excess body fat and/or excess body weight, insomnia, sleep disturbances, night-time awakenings, anxiety and depression, urinary symptoms of urgency, VMS and dysuria.

Examples of further preferred diseases and preferred sex hormone-dependent diseases according to the invention in men include, but are not limited to, benign prostatic hyperplasia (BPH), metastatic prostatic carcinoma, testicular cancer, breast cancer, androgen dependent acne, seborrhoea, hypertrichosis, male pattern baldness and in boys precocious puberty. xamples of such disease and preferred sex hormone-dependent disease according to the invention in women include, but are not limited to, endometriosis, abnormal puberty, uterine fibrosis, uterine fibroid tumor, heavy menstrual bleeding, dysfunctional uterine bleeding, hormone-dependent cancers (breast, endometrial, ovarian, uterine), hyperandrogenism, hirsutism, hypertrichosis, female androgenetic alopecia, androgen dependent acne, seborrhoea, virilization, polycystic ovary syndrome (PCOS), premenstrual dysphoric disease (PMDD), HAIR-AN syndrome (hyperandrogenism, insulin resistance and acanthosis nigricans), ovarian hyperthecosis (HAIR-AN with hyperplasia ofluteinized theca cells in ovarian stroma), other manifestations of high intraovarian androgen concentrations ( e.g. follicular maturation arrest, atresia, anovulation, dysmenorrhea, dysfunctional uterine bleeding, infertility) and androgen-producing tumor (virilizing ovarian or adrenal tumor), and osteoporosis, as well as symtpoms associated therewith. Preferred are VMS associated with prostate cancer; benign prostatic hyperplasia (BPH), and/or metastatic prostatic carcinoma.

Several compounds identified as metabolites of elinzanetant were tested for their binding affinity and functional antagonistic activities to human NK1 and NK3 receptors in receptor binding assay formats and cellular functional assay formats.

As used herein, the term "hot flushes" is interchangeable with the term "hot flashes" and with the term "vasomotor symptoms" and intended to have the same meaning.

FIGURE LEGEND

**Figure 1:** Purification of 2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide a) TLCs of Example 4, Batch 3; and b) TLCs of Example 4, Batch 4.

**Figure 2:** IR spectrum of Example 4, Batch 4.

**Figure 3:** $^1$H-NMR spectrum of Example 4 at 25°C, Batch 4

**Figure 4:** $^1$H-NMR spectrum of Example 4 at 70°C, Batch 4

**Figure 5:** $^1$H-13C-HSQC NMR spectrum of Example 4 at 250°C, Batch 4

**Figure 6:** $^1$H-NOE NMR spectrum of Example 4 at 25°C, Batch 4

**Figure 7:** $^1$H-NOE NMR spectrum of Example 4 at 45°C, Batch 4

**Figure 8:** Full scan mass spectrum in positive ion mode of Example 4, Batch 4

**Figure 9:** MS/MS fragmentation mass spectrum in positive ion mode of the ion m/z 685 of Example 4, Batch 4

## General Procedures

The compounds according to the invention can be prepared according to the following Schemes 1 through 3.

The schemes and procedures described below illustrate synthetic routes to the compounds of general formula (I) of the invention and are not intended to be limiting. It is obvious to the person skilled in the art that the order of transformations as exemplified in the schemes can be modified in various ways. The order of transformations exemplified in the schemes is therefore not intended to be limiting. These modifications can be such as the introduction of protecting groups, cleavage of protecting groups, reduction or oxidation of functional groups, dehydrogenation, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art.

Specific examples are described in the subsequent paragraphs.

**Scheme 1**

Scheme for the synthesis of the compound of formula (V)

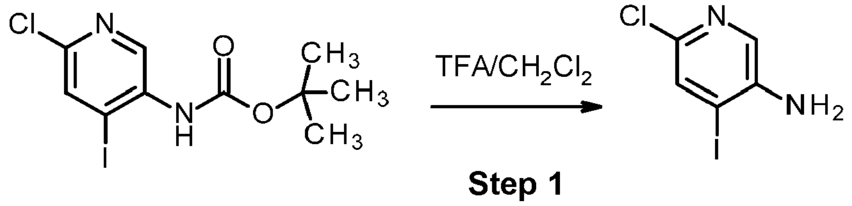


2B

2A

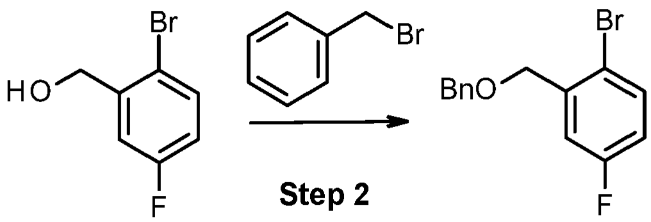


4C

4B

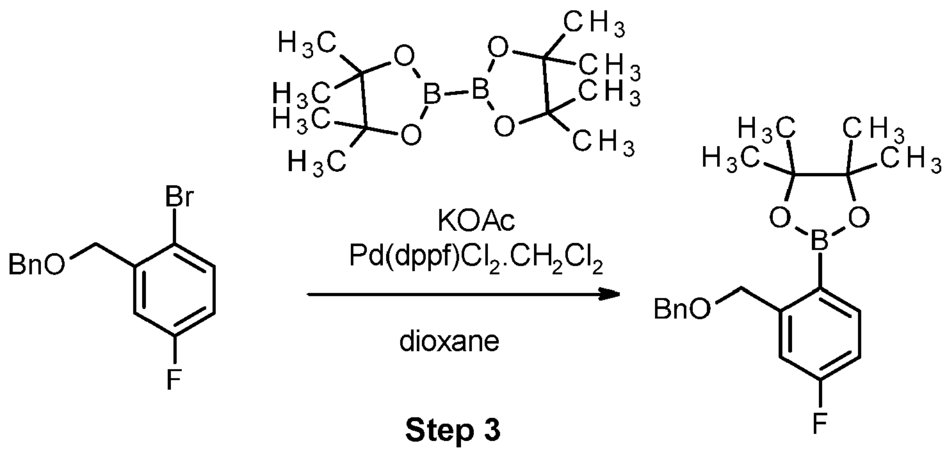


4B

4A-1

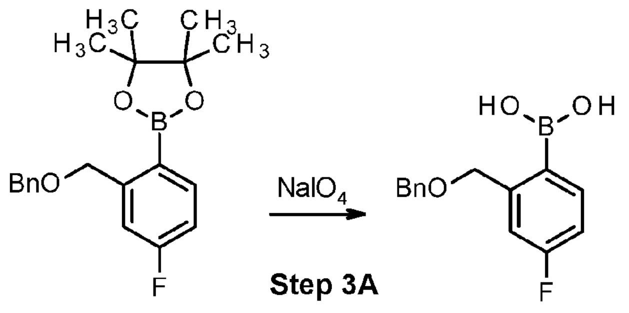


**4A-1** **4A**

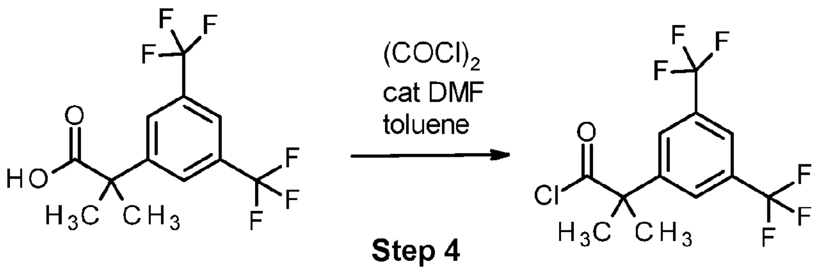


**1** **2**

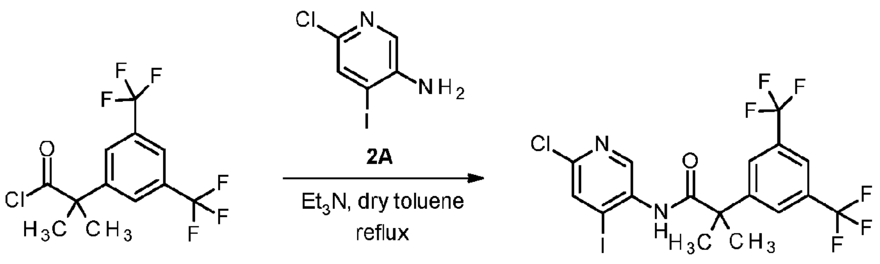


**2** **Step 5** **3**

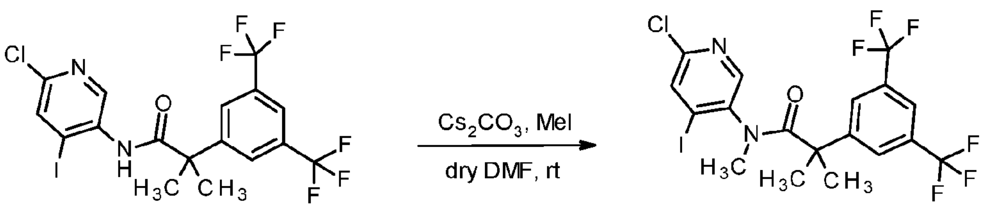


**3** **Step 6** **4**

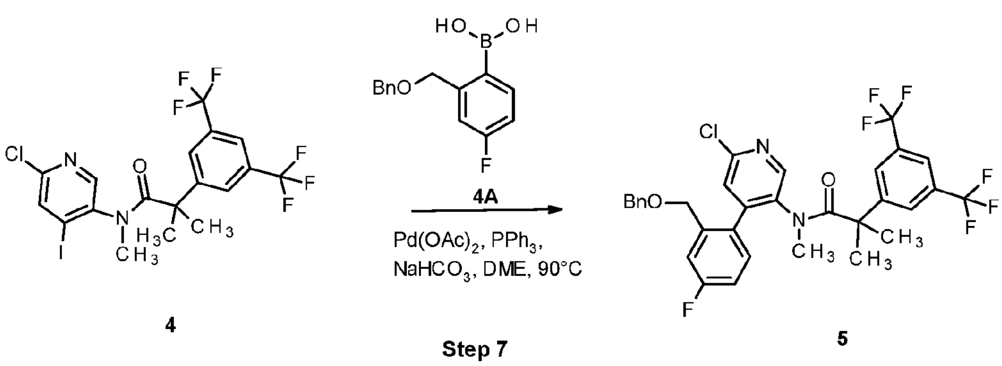


**4** **Step 7** **5**

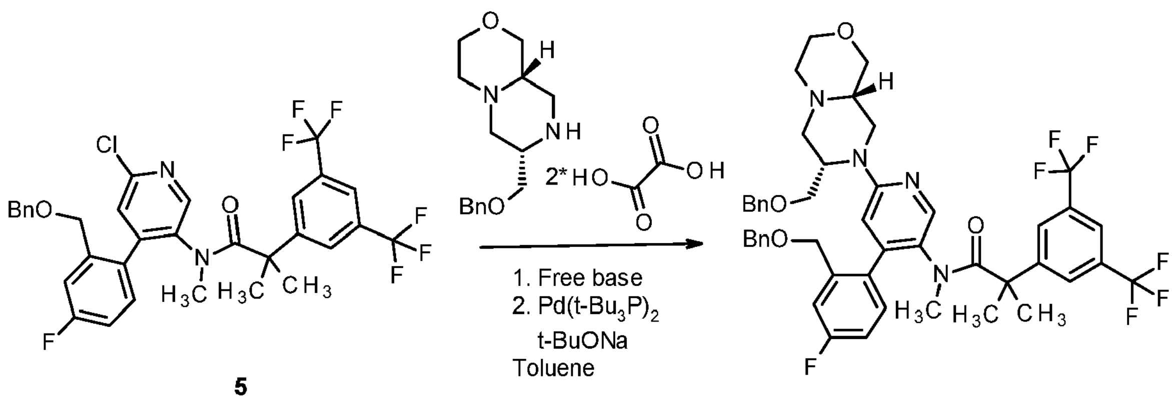
2* 
1. Free base
2. Pd(t-Bu$_3$P)$_2$
t-BuONa
Toluene

5

**Step 8**

6

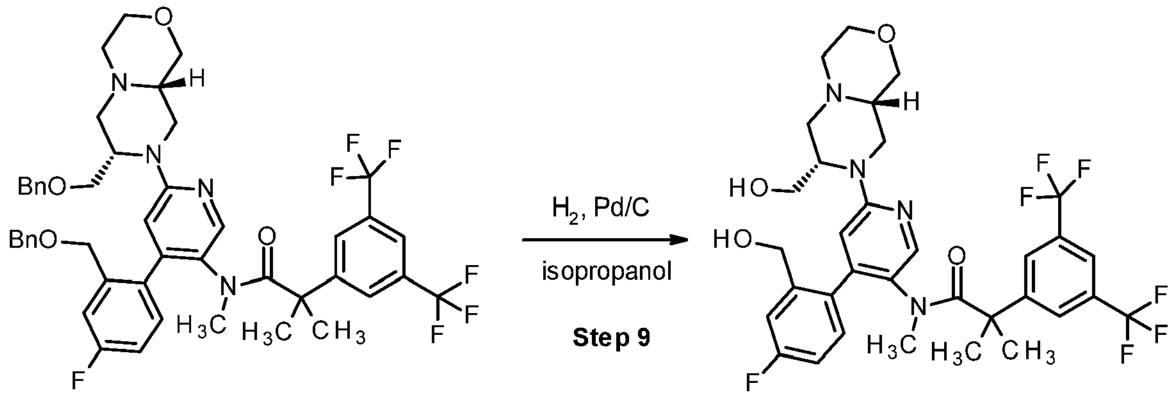
H$_2$, Pd/C
isopropanol
Step 9

6

V

**Scheme 2**

Scheme for the synthesis of the compound of formula (IV)

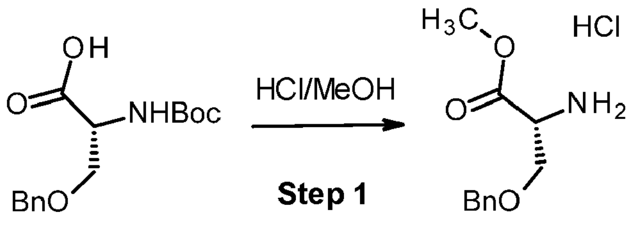


**A3-2** **A3-1**

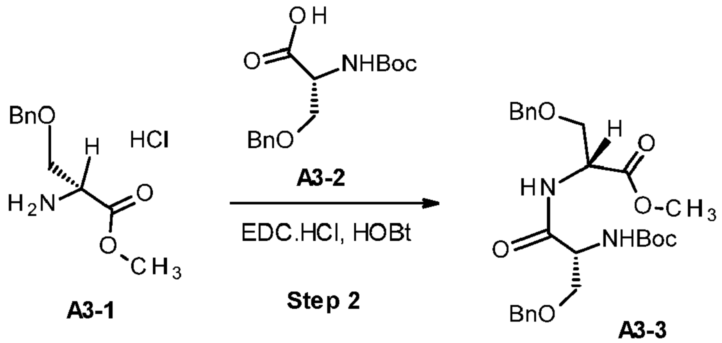


**A3-1** **A3-2** **A3-3**

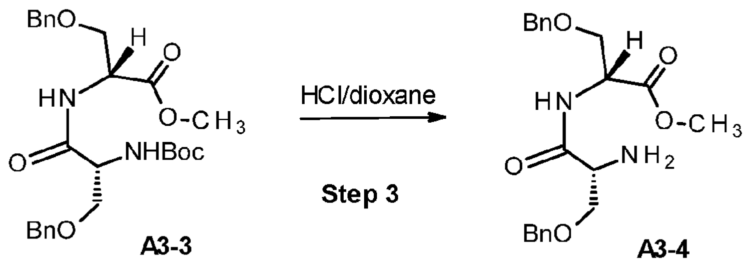

A3-3 A3-4

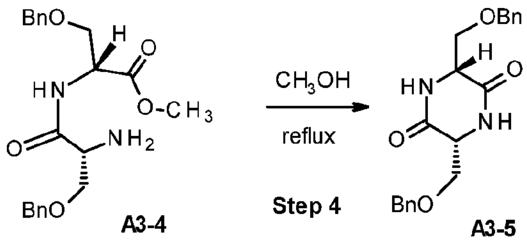

A3-4 A3-5

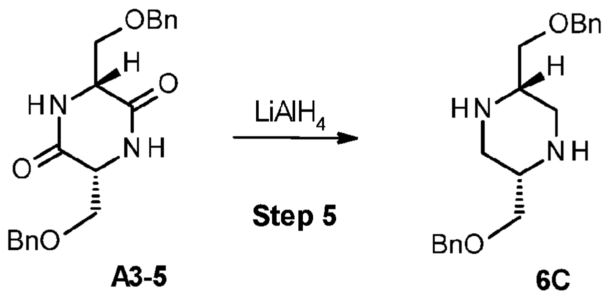

A3-5 6C

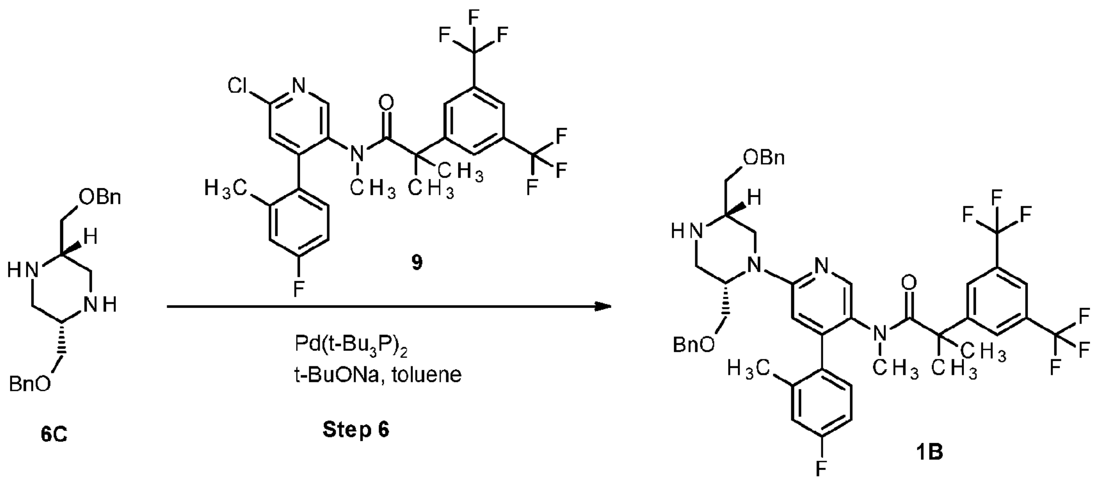

9 6C 1B

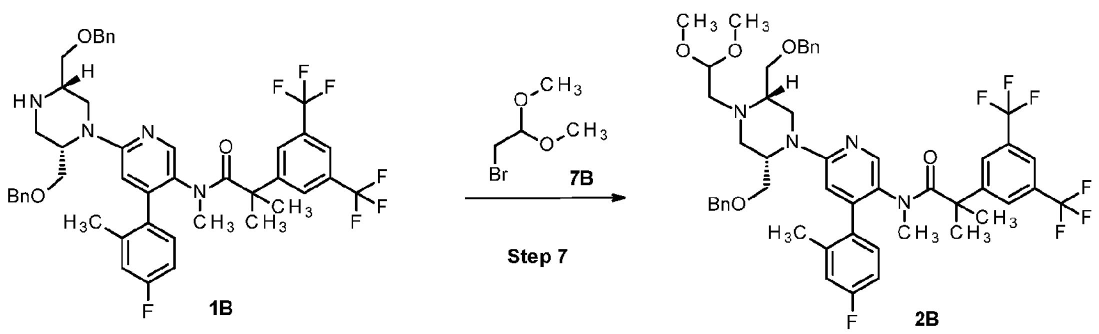

7B 1B 2B

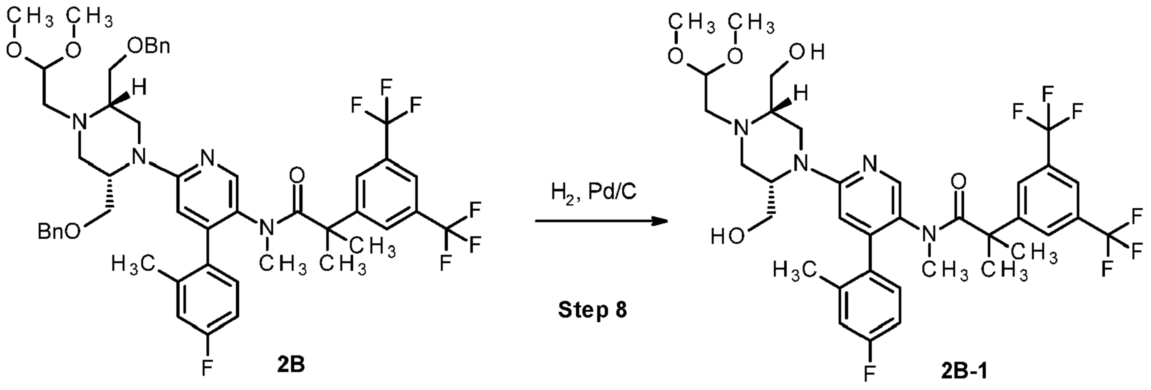


2B 2B-1

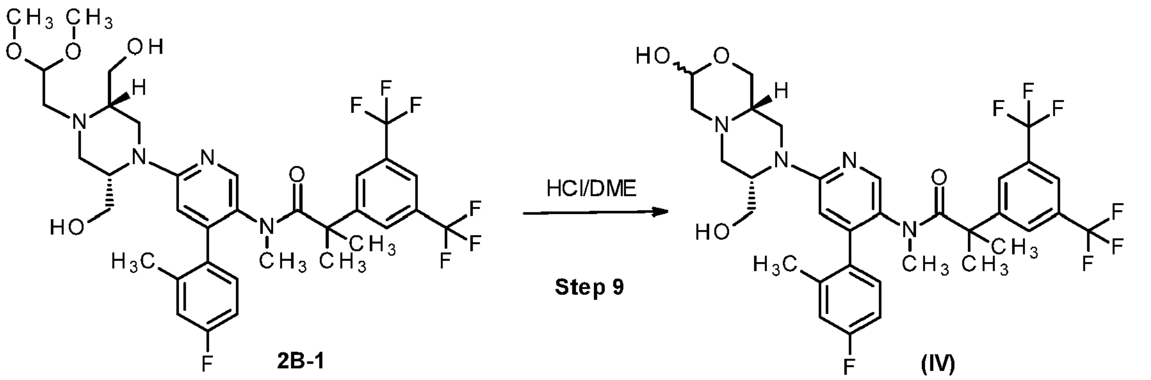


2B-1 (IV)

## Scheme 3

Scheme for the synthesis of the compound of formula (III)

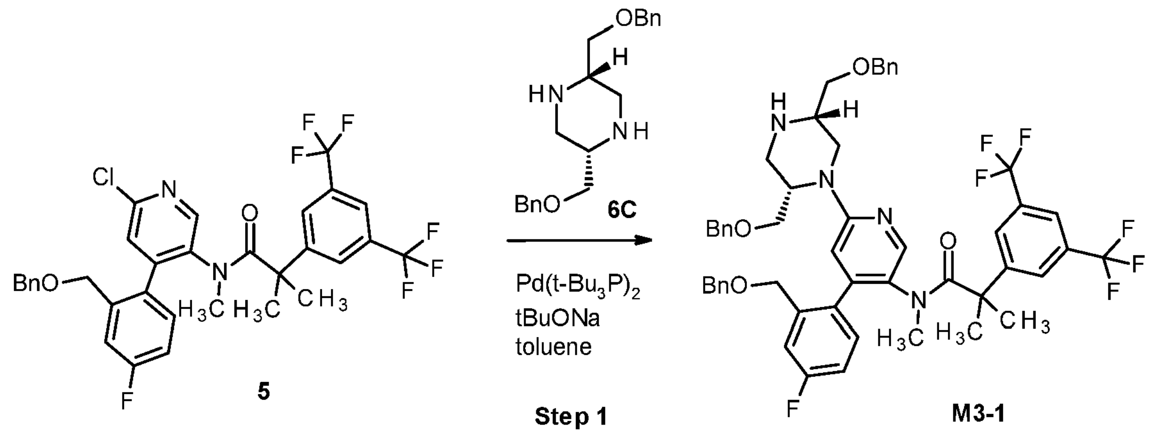


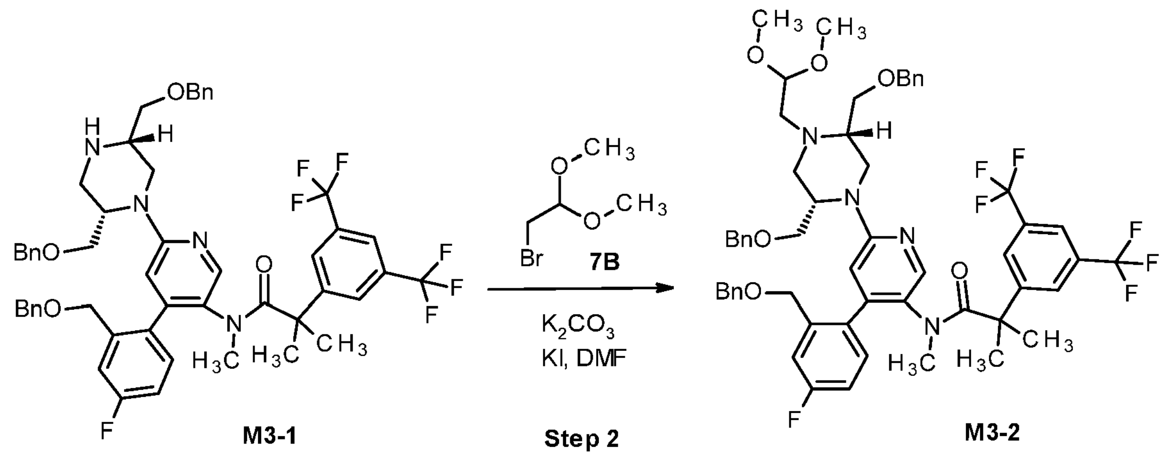

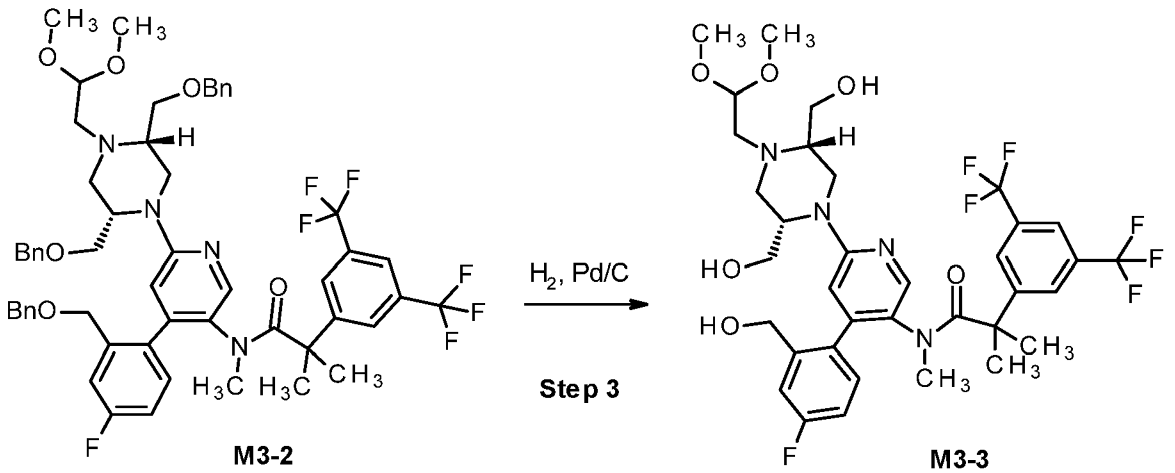


M3-2 M3-3

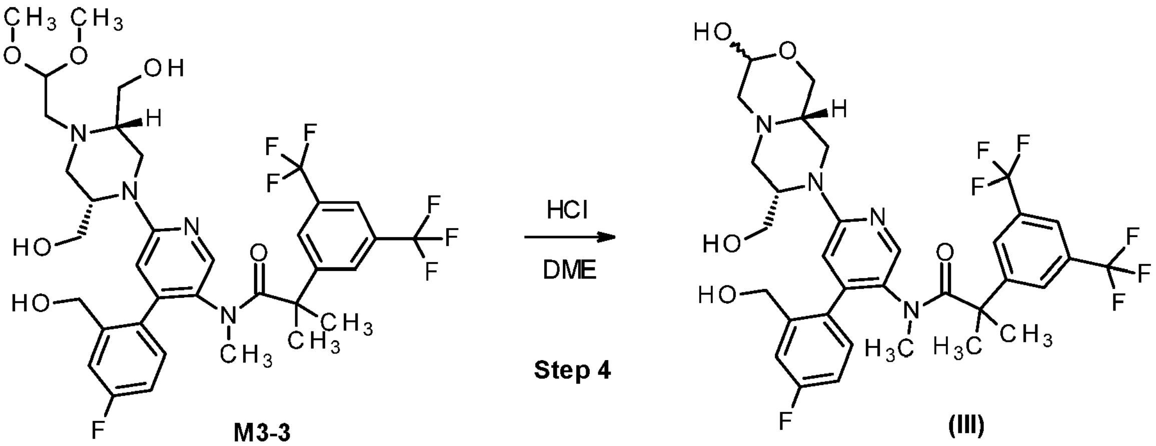


M3-3 (III)

EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

**Abbreviations**

| Abbreviation | Meaning |
|---|---|
| API | Active Pharmaceutical Ingredient |
| aq. | aqueous |
| Bn | Benzyl |
| Boc | tert- Butoxycarbonyl |
| $Cs_2CO_3$ | Cesium carbonate |
| Cu(I)Cl | Copper(I) chloride |
| ca. | circa |
| DCE | 1,2-Dichloroethane |
| DCM | Dichloromethane |
| DIAD | Diisopropyl azodicarboxylate |
| DIPEA | N-Ethyl-N-isopropylpropan-2-amine |
| DMAP | N,N-Dimethylpyridin-4-amine |
| DME | 1,2-Dimethoxyethane |
| DMF | N,N-Dimethylformamide |
| DMSO | Dimethyl sulfoxide |
| DP | Desired product |
| EDC | 3-(Ethyliminomethyleneamino)-*N*,*N*-dimethylpropan-1-amine |
| EE | Ethyl acetate |
| h | Hour |
| HATU | N-[(Dimethylamino)(3H-[1,2,3]triazolo[4,5-b]pyridin-3-yloxy)methylene]-N-methylmethanaminium hexafluorophosphate |
| HBr | Hydrogen bromide |
| HCl | Hydrochloric acid |
| hex | n-Hexane |
| HOBt | Hydroxybenzotriazole (Benzotriazol-1-ol) |
| HPLC | High performance liquid chromatography |
| $HNO_3$ | Nitric acid |
| $H_2SO_4$ | Sulfuric acid |
| Int | Intermediate |
| IPC | In process check |
| $K_2CO_3$ | Potassium carbonate |
| LC-MS | liquid chromatography - mass spectrometry |
| LCMS | liquid chromatography - mass spectrometry |
| LiOH | Lithium hydroxide |
| M | Molar |
| μW | Microwave |

(continued)

| Abbreviation | Meaning |
|---|---|
| MeCN | Acetonitrile |
| MeI | Iodomethane ($CH_3I$) |
| MeOH | Methanol |
| $MgSO_4$ | Magnesium sulfate |
| min | Minute(s) |
| N | Normal |
| $Na_2CO_3$ | Sodium carbonate |
| NaH | Sodium hydride |
| $NaHCO_3$ | Sodium bicarbonate |
| NaI | Sodium iodide |
| NaOH | Sodium hydroxide |
| $Na_2SO_4$ | Sodium sulfate |
| $NH_4Cl$ | Ammonium chloride |
| NMR | nuclear magnetic resonance spectroscopy |
| $PdCl_2(PPh_3)_2$ | Bis(triphenylphosphine)palladium(II) dichloride |
| $Pd(dppf)Cl_2$ | [1,1'-Bis(diphenylphosphino)ferrocene]dichloropalladium(II) |
| $Pd(dppf)Cl_2 \cdot CH_2Cl_2$ | 1,1'-Bis(diphenylphosphino)ferrocene-palladium(II)dichloride dichloromethane complex |
| $Pd(OAc)_2$ | Palladium(II) acetate |
| $Pin_2B_2$ | 4,4,4',4',5,5,5',5'-Octamethyl-2,2'-bi(1,3,2-dioxaborolane) |
| $PPh_3$ | Triphenylphosphine |
| ppm | parts per million |
| Py | Pyridine |
| RT | Room temperature |
| rt | Retention time |
| Rt | Retention time |
| sat. | Saturated |
| SEM | 2-(trimethylsilyl)ethoxymethyl |
| SFC | Solid Phase Chromatography |
| SM | Starting material |
| STAB | Sodium triacetoxyborohydride |
| $HSnBu_3$ | Tributyltin hydride |
| TLC | Thin Layer Chromatography |
| TMS-azide | Azidotrimethylsilane |
| $TMS-N_3$ | Azidotrimethylsilane |
| T3P | Propylphosphonic anhydride |
| TBAB | Tetra-N-butylammonium bromide |

(continued)

| Abbreviation | Meaning |
|---|---|
| TBAI | Tetra-N-butylammonium iodide |
| TBME | tert-Butyl methyl ether |
| TEA | Triethylamine |
| TFA | Trifluoroacetic acid |
| $(Tf)_2O$ | Trifluoromethanesulfonic anhydride |
| TfO- | Trifluoromethanesulfonate |
| THF | Tetrahydrofuran |
| TLC | Thin layer chromatography |
| %a | %area by HPLC |

**Analysis methods**

**HPLC methods**

Method 1:For compound of formula (III)

<table>
<tr><td>Column</td><td colspan="2">Xtimate C18 2.1*30 mm, 3 μm</td></tr>
<tr><td rowspan="8">Mobile Phase</td><td colspan="2">A:Water (4L)+TFA (1.5mL)</td></tr>
<tr><td colspan="2">B:Acetonitrile (4L)+TFA (0.75mL)</td></tr>
<tr><td>Time (min)</td><td>B%</td></tr>
<tr><td>0</td><td>10</td></tr>
<tr><td>6.0</td><td>80</td></tr>
<tr><td>7.0</td><td>80</td></tr>
<tr><td>7.01</td><td>10</td></tr>
<tr><td>8.0</td><td>10</td></tr>
<tr><td>Flow Rate</td><td colspan="2">1.0 mL/min</td></tr>
<tr><td>Wavelength</td><td colspan="2">PDA 220 nm, 215 nm, 254 nm</td></tr>
<tr><td>Oven Temp</td><td colspan="2">40 °C</td></tr>
<tr><td>Instrument</td><td colspan="2">Shimadzu LC-20AB</td></tr>
</table>

**Description:**

Mobile phase: Ramp from 10% ACN (0.018%TFA) in water (0.037%TFA) to 80% ACN in water in 4.5 min; then hold at 80% ACN for 1.5 minutes; return back to 10% ACN in water within 0.5 min and hold for 1.5 min. Flow rate is set at 1.2ml/min, column temperature at 40°C and detector wavelength at 220 nm, 215 nm and 254 nm. The column is of Xtimate C18 2.1*30mm,3 μm.

Method 2:

For compound of formula V

| | | |
|---|---|---|
| **Instrument Name** | Agilent 1260 HPLC system equipped with DAD detector | |
| **Column** | XBridge® C18 3.0*150 mm, 3.5 μm | |
| **Mobile phase** | **A**: 0.05% $NH_3 \cdot H_2O$ (v/v) in water;<br>**B**: ACN | |
| **Inj. Volume (μL)** | 5.0 | |
| **Wavelength/Bandwidth** | 220 nm/4 nm | |
| **Acquisition Time(min)** | 15 | |
| **Post Time(min)** | 5 | |
| **Flow Rate(ml/min)** | 1.0 | |
| **Column Tem. (°C)** | 40 | |
| **Gradient** | **Time (min)** | **B%** |
| | 0.00 | 30 |
| | 15.00 | 95 |

Method 3:

For compound of formula IV

| | | |
|---|---|---|
| **Instrument** | Agilent 1260 HPLC system equipped with DAD detector | |
| **Column** | YMC-Pack ODS-A 150*4.6 mm, DS-5 μm, 12 nm | |
| **Mobile Phase** | **A:** 0.04% TFA (v/v) in water | |
| | **B:** 0.02% TFA (v/v) in ACN | |
| **Inj. Volume (μL)** | 10.0 | |
| **Wavelength/Bandwidth** | 220 nm/4 nm | |
| **Acquisition Time(min)** | 15 | |
| **Post Time(min)** | 5 | |
| **Flow Rate(ml/min)** | 1.0 | |
| **Column Tern. (*C)** | 40 | |
| **Gradient** | **Time (min)** | **B%** |
| | 0.00 | 5 |
| | 8.00 | 95 |
| | 15.00 | 95 |

Method 4:

Solid Phase Chromatography for intermediate for Synthesis of compound of formula IV

| | |
|---|---|
| Column | Chiral MD-3, 100 A, 4.6mm, 3um |

(continued)

| | | |
|---|---|---|
| Mobile Phase | A: CO2 | |
| | B:ethanol (0.05% DEA) | |
| | Time (min) | B% |
| | 0 | 5 |
| | 4.0 | 40 |
| | 6.5 | 40 |
| | 8.0 | 5 |
| Flow Rate | 2.8 mL/min | |
| Wavelength | PDA 220 nm | |
| Oven Temp | 35 °C | |
| Instrument | Waters UPCC with PDA Detector and QDa Detector | |

## EXAMPLES

**Example 1: 2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide (compound of formula V) in accordance with Scheme 1, *supra***

***Step 1: Preparation of Intermediate 2A***

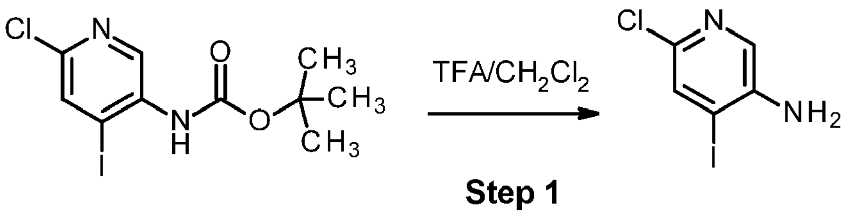


**2B** **2A**

Two batches were carried out.

To a solution of tert-butyl N-(6-chloro-4-iodo-3-pyridyl)carbamate (intermediate 2B) (CAS No.400777-00-6; 700 g, 1.97 mol, 1.00 eq) in DCM (1.4 L) was added TFA (2.16 kg, 18.91 mol, 1.40 L, 9.58 eq) slowly at 0°C, after addition, the mixture was stirred at 15°C for 12 h. LCMS analysis showed the intermediate 2B was consumed. The two batches were combined for work-up. The mixture was basified with saturated aqueous $Na_2CO_3$ to pH = 7-8, then extracted with EtOAc (3 L× 3). The combined organic layers were washed with brine (3 L), dried over $Na_2SO_4$, filtered and concentrated under reduced pressure to give a residue. The mixture was triturated with (Petroleum ether: Ethyl acetate = 30:1, 3.1 L). Intermediate 2A (950 g, 3.73 mol, 94.56% yield) was obtained as a yellow solid analysed by $^1$H-NMR. **LCMS:** Column Xtimate C18,3μm,2.1*30mm RT = 0.767 min, M+H = 255.0, purity: 92% **$^1$H-NMR:** (400 MHz, $CDCl_3$), δ = 7.83 - 7.78 (m, 1H), 7.60 (s, 1H), 4.29 - 4.05 (m, 2H)

***Step 2: Preparation of intermediate 4B***

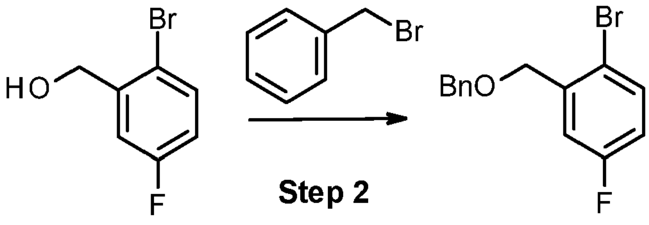


4C 4B

Two batches were carried out.

To a solution of (2-bromo-5-fluoro-phenyl)methanol (intermediate 4C) (250 g, 1.22 mol, 1.00 eq) in DMF (2 L) was added NaH (97.54 g, 2.44 mol, 60% purity, 2.00 eq) in portions at 0°C. The mixture was stirred for 30 mins at 0°C. To the mixture was added BnBr (229.41 g, 1.34 mol, 159.31 mL, 1.10 eq) and then the mixture was stirred at 20°C for 12 h. TLC (Petroleum ether: Ethyl acetate = 10:1, $R_f$=0.36) showed the intermediate 4C was consumed. The two batches were combined for work-up. The mixture was poured into 20 L water and then the solution was extracted with Ethyl acetate (5 L× 2). The combined organic layers were concentrated under reduced pressure to give a crude product. Intermediate 4B (730 g, crude) was obtained as a yellow oil analysed by $^1$HNMR.

**$^1$H-NMR:** (400 MHz, $CDCl_3$), δ = 7.49 (dd, J=5.2, 8.7 Hz, 1H), 7.45 - 7.37 (m, 4H), 7.37 - 7.30 (m, 2H), 6.89 (dt, J=3.1, 8.3 Hz, 1H), 4.68 (s, 2H), 4.60 (s, 2H)

***Step 3: Preparation of intermediate 4A-1***

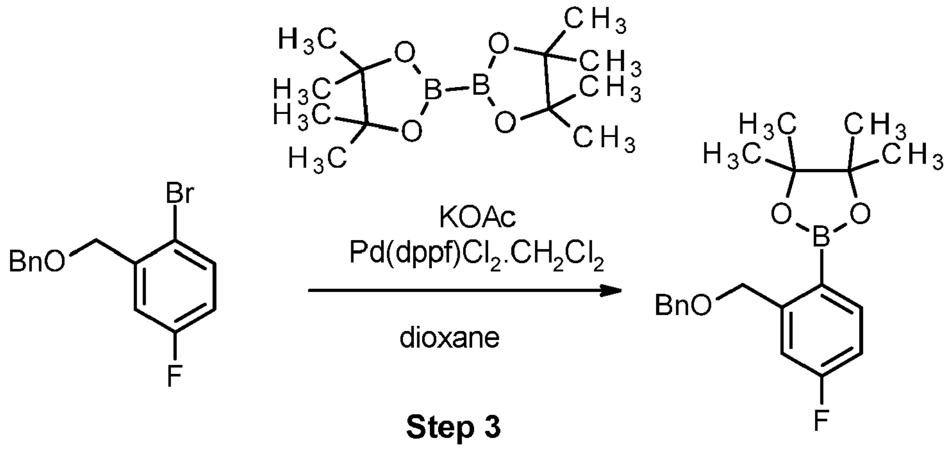


4B 4A-1

To a solution of intermediate 4B (700 g, 2.37 mol, 1.00 eq) in dioxane (3.5 L) was added $Pin_2B_2$ (632.38 g, 2.49 mol, 1.05 eq), KOAc (465.53 g, 4.74 mol, 2 eq), and $Pd(dppf)Cl_2 \cdot CH_2Cl_2$ (38.74 g, 47.43 mmol, 0.02 eq), then the mixture was stirred at 100°C for 12 h under $N_2$. TLC (Petroleum ether: Ethyl acetate = 10:1, $R_f$= 0.33) showed intermediate 4B was consumed. The mixture was filtered and the filtrate was concentrated in vacuum to give a crude product. The crude product was purified by flash silica gel chromatography (Petroleum ether: Ethyl acetate = 5-10%). Intermediate 4A-1 (750 g, crude) was obtained as a green solid analysed by $^1$HNMR.

**$^1$H-NMR:** (400 MHz, $CDCl_3$),δ = 7.81 (dd, J=6.7, 8.1 Hz, 1H), 7.46 - 7.36 (m, 5H), 7.34 - 7.26 (m, 3H), 7.02 - 6.91 (m, 1H), 4.86 (s, 2H), 4.63 (s, 2H), 1.32 (s, 12H)

***Step 3A: Preparation of intermediate 4A***

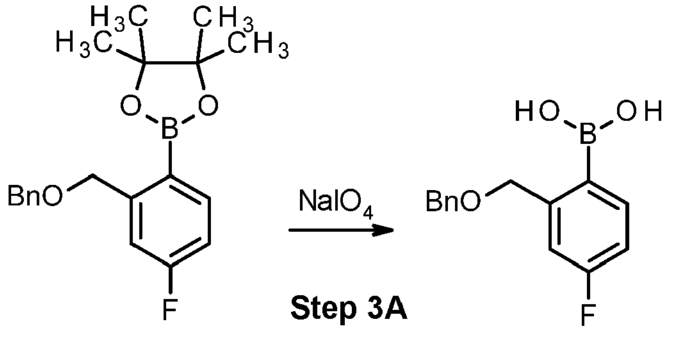


**4A-1** **4A**

Three batches were carried out.

To a solution of intermediate 4A-1 (250 g, 730.54 mmol, 1.00 eq) in acetone (2 L) at 15°C was added sodium periodate (468.77 g, 2.19 mol, 121.44 mL, 3.00 eq) and $NH_4OAc$ solution (1 M, 1.46 L, 2.00 eq), the mixture was stirred for 3 h. More water was added (500 mL), and the mixture was stirred at 40° C for 2 h. After addition of HCl (3 M, 487.45 mL, 2.00 eq), the mixture was stirred at 15°C for 2 h. TLC (Petroleum ether: Ethyl acetate = 3:1, $R_f$ = 0.66) showed the intermediate 4A-1 was consumed. Three batches were combined for work-up. The reaction mixture was filtered and concentrated under reduced pressure to give an aqueous solution, which was extracted with EtOAc (3L × 2). The combined organic layers were washed with brine (2 L), dried over $Na_2SO_4$, filtered and concentrated under reduced pressure to give a residue. The crude product was triturated with Petroleum ether (1.5 L) at 15° C for 12 h. Intermediate 4A (405 g, 1.56 mol, 71.06% yield) was obtained as a white solid analysed by $^1$HNMR.

**$^1$H-NMR:** (400 MHz, $CDCl_3$),δ = 7.77 (dd, J=6.6, 8.1 Hz, 1H), 7.33 - 7.17 (m, 4H), 7.07 - 6.84 (m, 2H), 6.52 - 6.43 (m, 1H), 4.80 (s, 2H), 4.56 (s, 1H), 4.50 (s, 2H), 4.47 (s, 1H)

***Step 4: Preparation of intermediate 2***

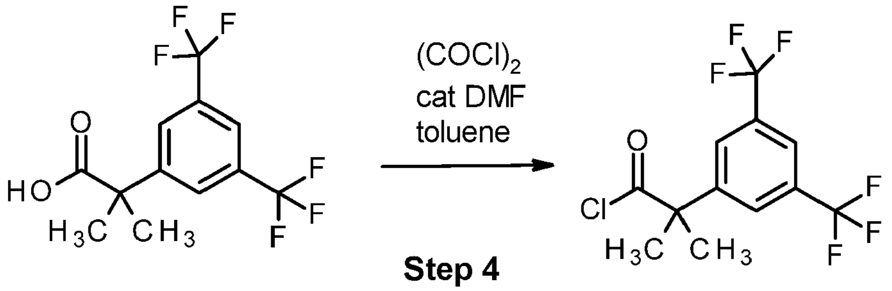


**1** **2**

Four batches were carried out.

2-[3,5-bis(trifluoromethyl)phenyl]-2-methyl-propanoic acid (intermediate 1) (CAS No. 289686-70-0; 420 g, 1.40 mol, 1.00 eq), DMF (5.11 g, 69.95 mmol, 5.38 mL, 0.05 eq) and toluene (3.5 L) were added to a reactor under $N_2$ at 25°C with stirring. $(COCl)_2$ (221.98 g, 1.75 mol, 153.09 mL, 1.25 eq) was then added drop-wise keeping the temp at ~25°C (slightly exothermic). At the end of the addition most of the solid had dissolved. The mixture was then allowed to stir at 25°C for 4 h. TLC (Petroleum ether: Ethyl acetate = 2: 1, $R_f$ = 0.47) showed the intermediate 1 was consumed. Four batches were combined for work-up. The mixture was concentrated in vacuum at 50°C to remove three-quarter of the toluene to give a crude product. The crude product was used for the next step without purification. Intermediate 2 was obtained as a toluene solution (3.5 L, yellow).

***Step 5: Preparation of intermediate 3***

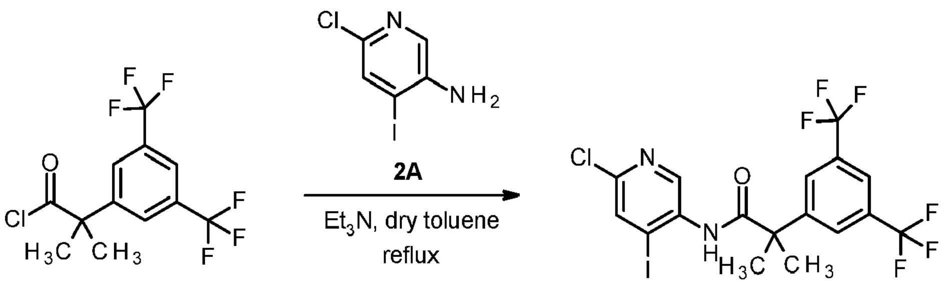


2 Step 5 3

Two batches were carried out in parallel.

To a solution of $Et_3N$ (188.89 g, 1.87 mol, 259.82 mL, 2.00 eq) and intermediate 2A (237.5 g, 933.37 mmol, 1.00 eq) in toluene (1.5 L) was added intermediate 2 (446.12 g, 1.40 mol, 1.50 eq) slowly below 40°C. The mixture was stirred at 110°C (reflux) for 4 h. LCMS showed intermediate 2A remained, the mixture was stirred at 110°C (reflux) for further 12 h. LCMS showed the intermediate 2A remained, the mixture was stirred at 110°C (reflux) for 12 h. LCMS showed that most of intermediate 2A was consumed. The two batches and a third batch were combined for work-up. The reaction mixture was cooled to 20°C and water (1.2 L) was carefully added. The phases were separated, and the aqueous layer extracted with toluene (300 mL × 2). The combined organic phases were washed successively with 3M HCl (800 mL), 7% aq sodium bicarbonate (800 mL), and water (1.5 L). The combined organic layers were dried over $MgSO_4$, filtered and the filtrate was concentrated in vacuum to give a crude product. The crude product was purified by trituration with Petroleum ether: Ethyl acetate (3.1 L 30:1) at 110°C for 12 h. Intermediate 3 (1.48 kg, 2.76 mol, 147.74% yield) was obtained as a yellow solid analysed by $^1$HNMR.

> **LCMS:** Column Chromolith Flash RP-18, 5μm,3.0*25mm, RT = 1.068 min, M+H = 536.9, purity: 51%
> **$^1$H-NMR:** (400 MHz, $CDCl_3$), δ = 9.08 - 9.03 (m, 1H), 7.94 (s, 2H), 7.88 (s, 1H), 7.70 (s, 1H), 7.27 (s, 1H), 7.07 (br s, 1H), 1.81 (s, 6H)

***Step 6: Preparation of intermediate 4***

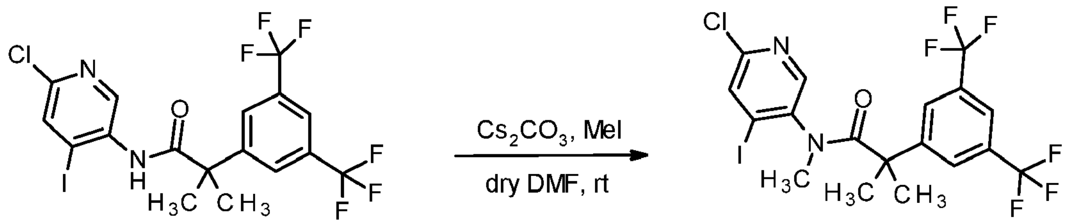


3 Step 6 4

Two batches were carried out in parallel.

Intermediate 3 (740 g, 1.38 mol, 1.00 eq) and $Cs_2CO_3$ (539.15 g, 1.65 mol, 1.20 eq) were suspended in DMF (3.5 L) at 20°C. MeI (254.45 g, 1.79 mol, 111.60 mL, 1.3 eq) was then added and the resulting mixture stirred at 20°C for 18 h. LCMS showed the intermediate 3 was consumed. The two batches were combined for work-up. The reaction mixture was quenched by addition of water 30 L at 0 °C, and then extracted with EtOAc (5L×3). The combined organic layers were washed with brine (5 L), dried over $Na_2SO_4$, filtered and concentrated under reduced pressure to give a residue. The crude product was purified by trituration with Petroleum ether: Ethyl acetate = 30:1(3.1 L) at 15 °C for 8 h. Intermediate 4 (1.05 kg, 1.91 mol, 69.14% yield) was obtained as a yellow solid analysed by $^1$HNMR.

> **LCMS:** Column Ultimate XB-C18, 3μm,3.0*50mm, RT = 6.004 min, M+H = 550.9, purity: 84%
> **$^1$H-NMR:** (400 MHz, $CDCl_3$), δ = 8.19 - 7.13 (m, 4H), 3.40 - 2.54 (m, 3H), 1.83 - 1.45 (m, 6H)

***Step 7: Preparation of intermediate 5***

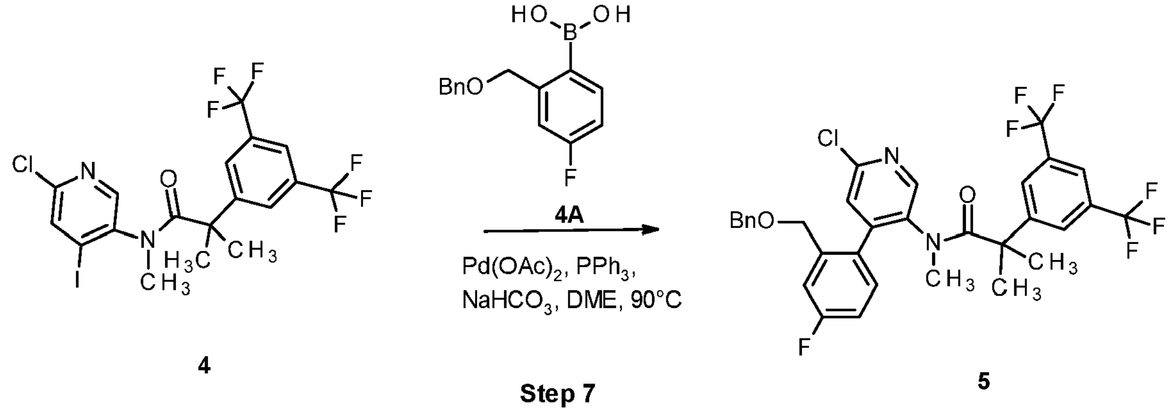


4

Step 7

5

To a solution of intermediate 4 (1.05 kg, 1.91 mol, 1 eq) in DME (10 L) was added intermediate 4A (495.90 g, 1.91 mol, 1 eq), $PPh_3$ (50.01 g, 190.68 mmol, 0.1 eq), the resulting mixture was stirred at 15°C for 10 minutes under $N_2$. $NaHCO_3$ (2 M, 1.91 L, 2 eq) was then added and the mixture stirred for a further 5 minutes. $Pd(OAc)_2$ (21.40 g, 95.34 mmol, 0.05 eq) was then added and the resulting suspension was heated at 90°C for 4 h. Additional intermediate 4A (20 g) was then added and the mixture stirred at 90°C for 12 h. LCMS showed the intermediate 4 was consumed completely. The reaction mixture was filtered, the organic layer was concentrated under reduced pressure to give a residue. The residue was purified by flash silica gel chromatography (Petroleum ether: Ethyl acetate = 5-10%) to afford a crude product with 85% LC purity (TLC Petroleum ether: Ethyl acetate 5:1, $R_f$ = 0.51) which was further purified by trituration with 3L Petroleum ether: Ethyl acetate (30: 1) at 15°C for 12 h. Pure intermediate 5 (1.03 kg, 1.61 mol, 84.53% yield) was obtained as a light-yellow solid analysed by $^1$HNMR.

LCMS: Column Xtimate C18, 3μm,2.1*30mm, RT = 5.543 min, M+H = 639.3, purity: 84% **$^1$H-NMR:** (400 MHz, $CDCl_3$), δ = 8.17 (s, 1H), 7.79 (s, 1H), 7.63 (s, 2H), 7.30 (s, 5H), 7.08 (br s, 4H), 4.41 (d, J=18.3 Hz, 2H), 4.37 - 4.07 (m, 2H), 2.76 - 2.15 (m, 3H), 1.61 - 1.35 (m, 6H)

***Step 8: Preparation and purification of intermediate 6***

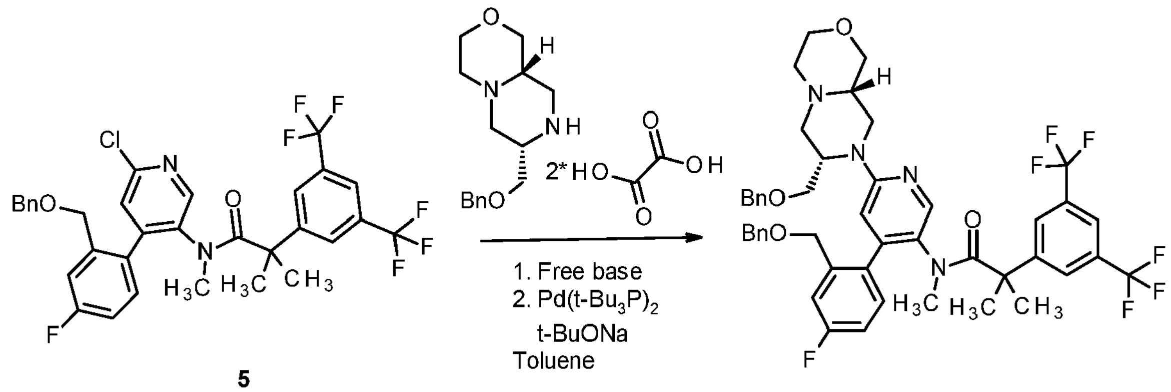


5

Step 8

6

Four batches were carried out.

(7S,9aS)-7-[(benzyloxy)methyl]octahydropyrazino[2,1-cl[1,4]oxazine dioxalic acid salt, as disclosed in WO 2011/023733 A1 as "Intermediate 3", (212.59 g, 603.28 mmol, 1.50 eq, dioxalate salt), toluene (20 L) and NaOH (1 M, 4.99 L, 12.4 eq) were charged to the vessel. The mixture was heated to 75°C and stirred for 5 min. The layers were settled for 30 min and the bottom aqueous layer discarded. The organic layer was cooled down to 25°C and water (10 L) was added. The mixture was stirred for 10 min, the layers settled for 15 min and the bottom aqueous layer discarded.

The organic layer was dried over $MgSO_4$, filtered, washed with toluene (1L×3), then concentrated under reduced pressure to give a solution of (7S,9aS)-7-[(benzyloxy)methyl]octahydropyrazino[2,1-cl[1,4]oxazine (10 L). In another reactor, intermediate 5 (257 g, 402.19 mmol, 1 eq), t-BuONa (77.30 g, 804.38 mmol, 2 eq) and $Pd(t-Bu_3P)_2$ (20.55 g, 40.22 mmol, 0.10 eq) were charged. The obtained solution of (7S,9aS)-7-[(benzyloxy)methyl]octahydropyrazino[2,1-cl[1,4]oxazine as prepared above was added and the transfer line rinsed with toluene (500 mL). The reaction mixture was heated to 85°C and stirred for 12 h before sampling for reaction completion. LCMS and TLC (Petroleum ether: Ethyl acetate = 2:1, $R_f$ = 2:1) showed the intermediate 5 was consumed. The four batches were combined for work-up. The mixture was concentrated under reduced pressure to give a residue. The residue was purified by flash silica gel chromatography (Petroleum ether: Ethyl acetate = 10-50%). Intermediate 6 (1.10 kg, 1.22 mol, 75.90% yield, 96% purity) was obtained as a yellow gum analysed by $^1$HNMR.

**LCMS:** Column Xtimate C18, 3μm,2.1*30mm, RT = 2.733 min, M+H = 865.5, purity: 65%
**$^1$H-NMR:** (400 MHz, $CDCl_3$), δ = 8.02 - 7.81 (m, 1H), 7.69 (s, 1H), 7.58 (s, 2H), 7.40 - 7.21 (m, 4H), 7.18 - 6.73 (m, 8H), 6.56 - 6.31 (m, 1H), 4.57 - 4.37 (m, 4H), 4.36 - 4.08 (m, 3H), 3.77 (d, J=10.9 Hz, 2H), 3.70 - 3.55 (m, 2H), 3.51 (d, J=5.0 Hz, 1H), 3.23 - 3.04 (m, 1H), 2.92 (t, J=11.3 Hz, 1H), 2.62 - 2.42 (m, 3H), 2.34 - 2.10 (m, 4H), 1.29 (d, J=3.5 Hz, 3H)

***Step 9: Preparation and purification* of 2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide (Compound of formula V)**

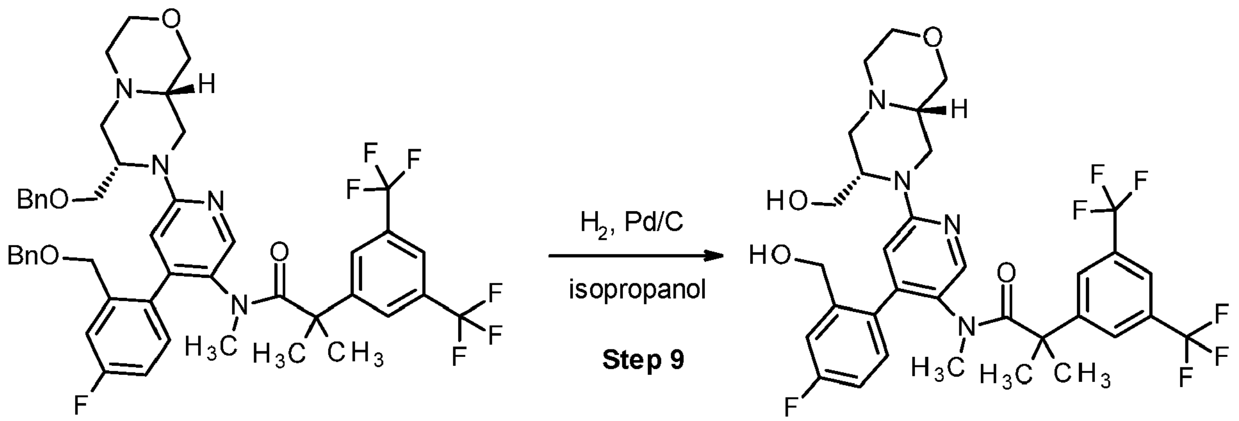


6 V

Five batches were carried out.

To a solution of intermediate 6 (100 g, 115.62 mmol, 1 eq) and HCl (44.37 g, 462.48 mmol, 43.50 mL, 38% purity, 4 eq) in isopropanol (1 L) was added Pd/C (20.00 g, 18.79 mmol, 10% purity). The mixture was heated to 50°C and stirred for 12 h under $H_2$ (40 Psi). LCMS showed the compound 6 was consumed. The five batches and another batch were combined for work-up. The suspension was filtered through a pad of Celite and the pad cake was washed with MeOH (1 L×2). The combined filtrates were concentrated to dryness to give a crude product. The crude was dissolved in 1L DCM, the pH was basified to 7-8 with sat. $NaHCO_3$ solution. The organic layer was separated, dried over anhydrous $Na_2SO_4$, filtered and the filtrate was concentrated in vacuum to give a crude product. The residue was purified by flash silica gel chromatography (Dichloromethane: Methanol = 2-10%). The compound of formula (V) (250 g, 361.68 mmol, 62.56% yield, 99.05% purity) was obtained as an off-white solid.

**LCMS:** Column Xtimate C18, 3um,2.1*30mm, RT = 1.708 min, M+H = 685.2, purity: 90%

Removal of residual solvent:

2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide (compound of formula V), as obtained above, (170 g, 248.30 mmol) was dissolved in $CH_3CN$ (170 mL), then $H_2O$ (340 mL) was added. Three batches were lyophilized to give a product. 2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide (501 g, 731.77

mmol, 98.24% yield) was obtained as an off-white solid analysed by $^1$HNMR.

**$^1$H-NMR:** (400 MHz, $CDCl_3$), $\delta$ = 7.93 (d, J=6.1 Hz, 1H), 7.79 (s, 1H), 7.60 (s, 2H), 7.43 - 7.30 (m, 1H), 6.97 (br d, J=5.9 Hz, 2H), 6.44 (br d, J=19.4 Hz, 1H), 5.30 (s, 1H), 4.69 - 4.41 (m, 2H), 4.40 - 4.30 (m, 1H), 4.17 - 3.65 (m, 8H), 3.39 - 3.28 (m, 1H), 3.14 - 2.95 (m, 2H), 2.76 (br d, J=11.3 Hz, 2H), 2.62 (br d, J=11.6 Hz, 2H), 2.47 - 2.26 (m, 2H), 2.01 (s, 1H), 1.94 - 1.38 (m, 3H), 1.33 (br s, 3H), 1.14 (s, 3H)

**Example 2: 2-[3,5-bis(trifluoromethyl)phenyl]-N-(4-(4-fluoro-2-methylphenyl)-6-[(7S,9aS)-7-(hydroxymethyl)-3-methylhexahydropyrazino[2,1-c][1,4]oxazin-8(1H)-yl]pyridin-3-yl}-N,2-dimethylpropanamide (compound of formula IV) according to Scheme 2, *supra***

***Step 1: Preparation of intermediate A3-1***

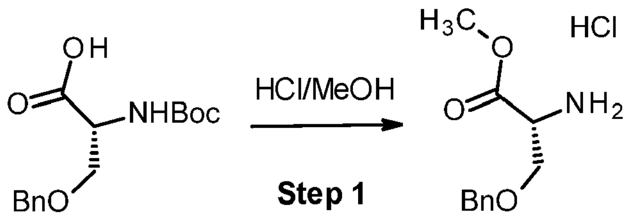


A3-2 A3-1

Two batches were carried out.

A mixture of (2R)-3-benzyloxy-2-(tert-butoxycarbonylamino)propanoic acid (intermediate A3-2) (CAS No. 47173-80-8; 250 g, 846.51 mmol, 1.00 eq) in HCl/MeOH (1 L) was stirred at 64 °C for 12 h. LCMS showed the starting material was consumed completely. The mixture was concentrated under reduced pressure to obtain intermediate A3-1 (416 g, crude) as a white solid, which was used for the next step directly.

**LCMS** Column Chromolith Flash RP-18, 5um,3.0*25mm, RT = 0.648 min, M+H = 210.2, purity: 92%

***Step 2: Preparation of intermediate A3-3***

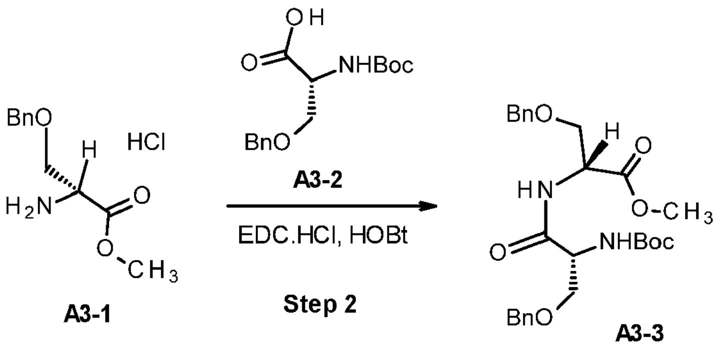


A3-1 A3-2 A3-3

To a mixture of intermediate A3-2 (250 g, 846.51 mmol, 1.00 eq), intermediate A3-1 (208 g, 846 mmol, 1.00 eq), EDC.HCl (324 g, 1.69 mol, 2.00 eq) HOBt (228 g, 1.69 mol, 2.00 eq) in DMF (2.5 L) was added DIPEA (547.03 g, 4.23 mol, 737.23 mL, 5.00 eq) dropwise at 0°C. The mixture was stirred at 20 °C for 12 h. TLC (Petroleum ether /EtOAc=3:1) and LCMS showed the starting material was consumed completely. The mixture was poured into $H_2O$ (2 L), the aqueous layer was extracted with $CH_2Cl_2$ (1 Lx3), the combined organic phases were washed with $H_2O$ (1 Lx3), brine (1 L) and concentrated under reduced pressure to obtain intermediate A3-3 (823 g, crude) as a light yellow oil, which was used for the next step directly.

LCMS Column Chromolith Flash RP-18, 5$\mu$m,3.0*25mm, RT = 1.002 min, M+H = 487.2, purity: 70%

***Step 3: Preparation of intermediate A3-4***

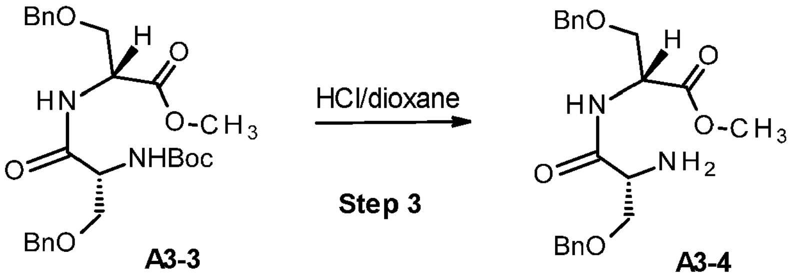


A solution of intermediate A3-3 (400 g, 822.10 mmol, 1.00 eq) in $CH_2Cl_2$ (2 L) and HCl/dioxane (4M, 1 L) was stirred at 20°C for 2 h. LCMS showed the starting material was consumed completely. The mixture was concentrated under reduced pressure to obtain intermediate A3-4 (634 g) as a yellow oil, which was used for the next step directly.

**LCMS** Column Chromolith Flash RP-18, 5μm,3.0*25mm, RT = 0.785 min, M+H = 387.2, purity: 69%

***Step 4: Preparation of intermediate A3-5***

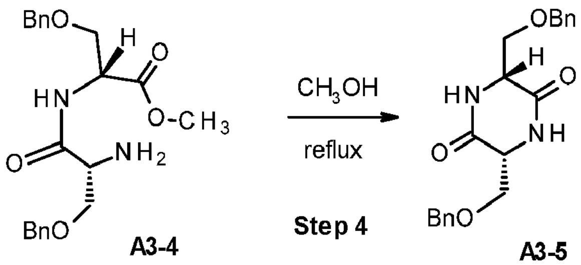


A mixture of intermediate A3-4 (315 g, 815.13 mmol, 1.00 eq) in MeOH (2.5 L) was stirred at 64 °C for 72 h. TLC (Petroleum ether: Ethyl acetate=3:1) showed the starting material was consumed completely. The mixture was concentrated under reduced pressure to obtain crude intermediate A3-5. The crude product was triturated with MeOH (50 mL) at 20°C for 30min to obtain intermediate A3-5 (370 g, 783.02 mmol, 48.03% yield, 75% purity) as a white solid analysed by LCMS, Chiral SFC, and $^1$H-NMR.

LCMS Column Chromolith Flash RP-18, 5μm,3.0*25mm, RT = 0.829 min, M+H = 355.2, purity: 83.8% at 254 nm.
**Chiral SFC:** Instrument CAS-QD-ANA-SFC-SE (Waters UPCC with PDA Detector and QDa Detector); Column: Chiral MD-3 100x4.6mm I.D., 3μm; Mobile phase: A: $CO_2$, B:ethanol (0.05% DEA); Gradient: from 5% to 40% of B in 4 min and hold 40% for 2.5 min, then 5% of B for 1.5 min; Flow rate: 2.8mL/min
Column temp.: 35°C; ABPR: 1500psi;
M+H= 355.2, M+Na= 377.2, Isomer 1 RT 3.56 min, 19.3%; Isomer 2 RT 3.92 min, 80.71%.(, RT 0.829 min)
**$^1$H-NMR:** (400 MHz $CDCl_3$), δ = 7.38-7.28 (m, 10H), 6.39 (s, 4H), 4.59-4.43 (m, 4H), 4.22 (d, *J* = 6.4 Hz, 2H), 3.87-3.84 (m, 2H), 3.71-3.67 (m, 2H).

***Step 5: Preparation of intermediate 6C***

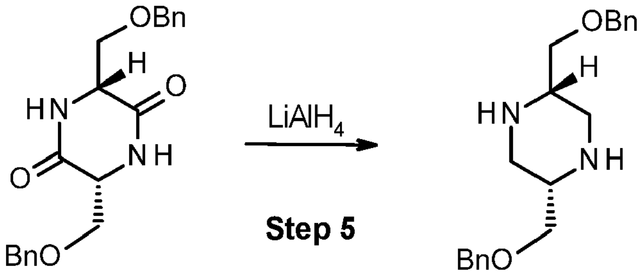


A3-5

6C

To a solution of intermediate A3-5 in THF (2.2 L) was added $LiAlH_4$ (36.63 g, 965.01 mmol, 3.00 eq) at 0°C, the mixture was stirred at 64°C for 12 h. TLC (Dichloromethane : Methanol = 10 : 1, $R_f$ = 0.78) showed the intermediate A3-5was consumed. The mixture was cooled to 0°C and quenched with $H_2O$ (40 mL). 10% Aqueous NaOH solution (40 mL) and $H_2O$ (120 mL) were added and the mixture was stirred at 20°C for 1 h. The mixture was filtered through celite and the filtrate was concentrated under reduced pressure to obtain the crude product. The residue was purified by flash silica gel chromatography (Dichloromethane: Methanol = 1-10%). Intermediate **6C** (56.0 g, 171.55 mmol, 53.33% yield) was obtained as brown oil analysed by $^1$HNMR.

**$^1$H-NMR:** (400 MHz, $CDCl_3$), δ = 7.43 - 7.21 (m, 9H), 4.58 - 4.51 (m, 4H), 3.71 - 3.60 (m, 2H), 3.48 (dd, J=4.4, 9.2 Hz, 2H), 3.12 - 3.02 (m, 2H), 2.91 - 2.83 (m, 2H), 2.83 - 2.74 (m, 2H), 2.28 ( s, 2H)

***Step 6: Preparation of intermediate 1B***

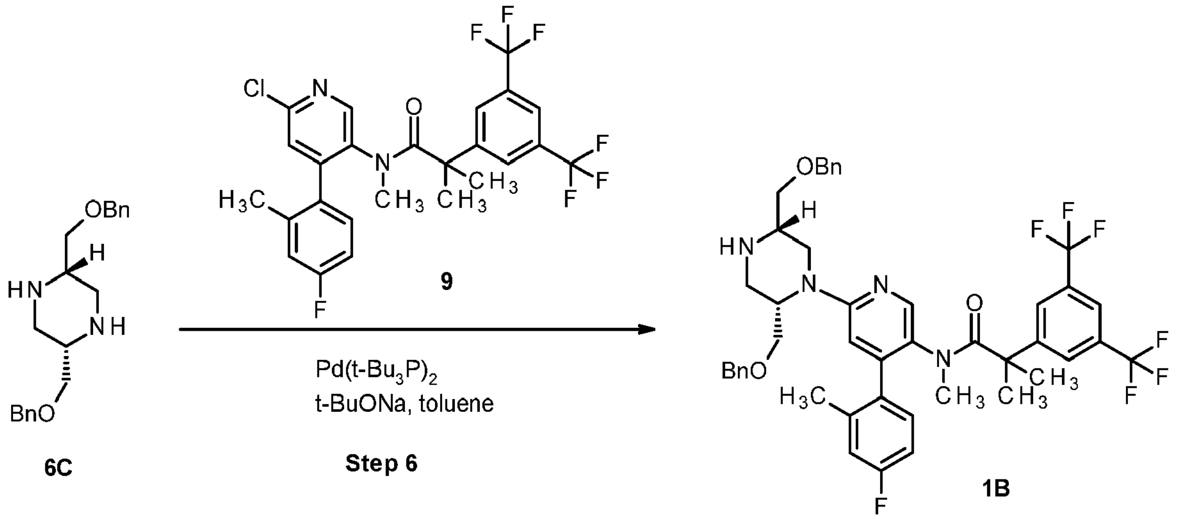


9

6C

1B

To a mixture of intermediate 6C (98.0 g, 300.22 mmol, 1.00 eq), 2-[3,5-bis(trifluoromethyl)phenyl]-N-[6-chloro-4-(4-fluoro-2-methyl-phenyl)-3-pyridyl]-N,2-dimethylpropanamide (intermediate 9) (as disclosed as Intermediate 4D in WO 2005/002577 A1, 161.58 g, 303.22 mmol, 1.01 eq) and t-BuONa (50.49 g, 525.38 mmol, 1.75 eq) in toluene (1 L) was added $Pd(t\text{-}Bu_3P)_2$ (15.34 g, 30.02 mmol, 0.10 eq). The mixture was stirred at 87°C for 12 h. LCMS and TLC (Dichloromethane: Methanol = 10: 1, $R_f$ = 0.21) showed the intermediate 6C was consumed. The mixture was concentrated under reduced pressure to obtain crude intermediate 1B. The crude material was purified by chromatography ($SiO_2$, Petroleum ether: Ethyl acetate = 30%-60%) to afford the product. Intermediate 1B (190 g) was obtained as a yellow solid analysed by $^1$H-NMR.

LCMS: Column Xtimate C18, 3μm,2.1*30mm, RT = 2.885 min, M+H = 823.5, purity: 64%

**$^1$H-NMR:** (400 MHz, $CDCl_3$), δ = 8.03 - 7.94 (m, 1H), 7.79 (s, 1H), 7.67 (s, 3H), 7.41 - 7.31 (m, 5H), 7.27 (s, 5H), 6.96 (s, 3H), 6.47 (s, 1H), 4.63 - 4.45 (m, 4H), 4.43 - 4.07 (m, 2H), 3.89 (s, 1H), 3.69 - 3.54 (m, 2H), 3.50 - 3.30 (m, 2H), 3.10 - 2.94 (m, 2H), 2.82 - 2.49 (m, 2H), 2.33 (s, 3H), 2.19 - 1.94 (m, 4H), 1.65 - 1.49 (m, 4H).

***Step 7: Preparation of intermediate 2B***

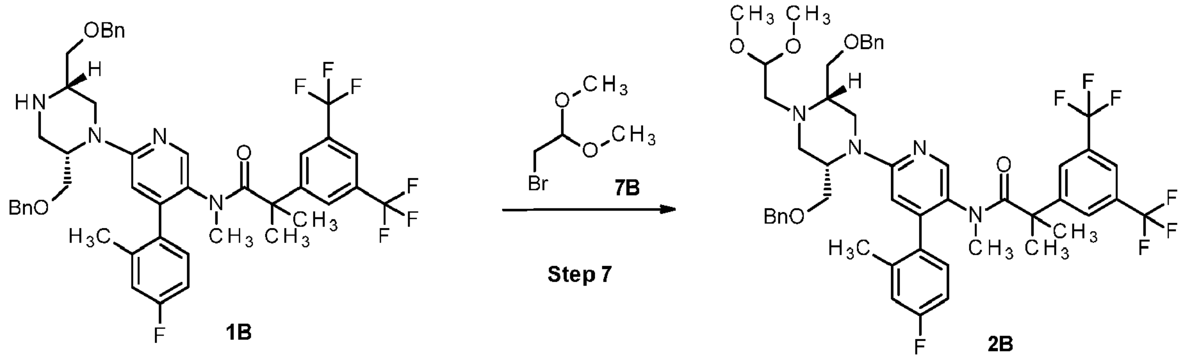


1B 2B

Four batches were carried out.

A mixture of intermediate 1B (47.5 g, 57.73 mmol, 1.00 eq), intermediate 7B (2-bromo-1,1-dimethoxy-ethane, CAS No. 7252-83-7, 19.51 g, 115.45 mmol, 13.55 mL, 2.00 eq), KI (1.92 g, 11.55 mmol, 0.20 eq), and $K_2CO_3$ (23.93 g, 173.18 mmol, 3.00 eq) in DMF (475 mL) was stirred at 100°C for 12 h. LCMS showed unreacted intermediate 1B remained, additional intermediate 7B (12.56 mL) was added and the mixture was stirred at 100°C for 48 h. A further LCMS showed the intermediate 1B was fully consumed. The four batches were combined for work-up. The mixture was poured into $H_2O$ (12 L) and extracted with EtOAc (2 Lx3), the combined organic phases were washed with brine (3 L), dried over anhydrous $Na_2SO_4$, and concentrated under reduced pressure to obtain the crude product. The residue was purified by flash silica gel chromatography (Petroleum ether: Ethyl acetate = 0-25%). Intermediate 2B (120 g, 131.73 mmol, 57.05% yield) was obtainedas a yellow solid analysed by $^1$H-NMR.

**LCMS:** Column Xtimate C18, 3μm,2.1*30mm, RT = 2.677 min, M+H = 911.4, purity: 71%

**$^1$H-NMR:** (400 MHz, $CDCl_3$), δ = 7.90 (s, 1H), 7.75 - 7.67 (m, 1H), 7.58 (s, 2H), 7.31 - 7.20 (m, 5H), 7.18 (d, J=2.1 Hz, 5H), 6.86 (s, 2H), 6.37 (s, 1H), 4.52 - 4.36 (m, 4H), 4.33 - 4.06 (m, 2H), 3.77 (br s, 1H), 3.62 - 3.48 (m, 2H), 3.46 - 3.36 (m, 1H), 3.29 - 3.15 (m, 6H), 3.02 - 2.92 (m, 1H), 2.97 (dd, J=5.4, 13.9 Hz, 1H), 2.85 - 2.72 (m, 1H), 2.58 (dd, J=4.1, 10.7 Hz, 1H), 2.52 - 2.35 (m, 2H), 2.33 - 1.87 (m, 5H), 1.49 - 1.21 (m, 5H)

***Step 8: Preparation of intermediate 2B-1***

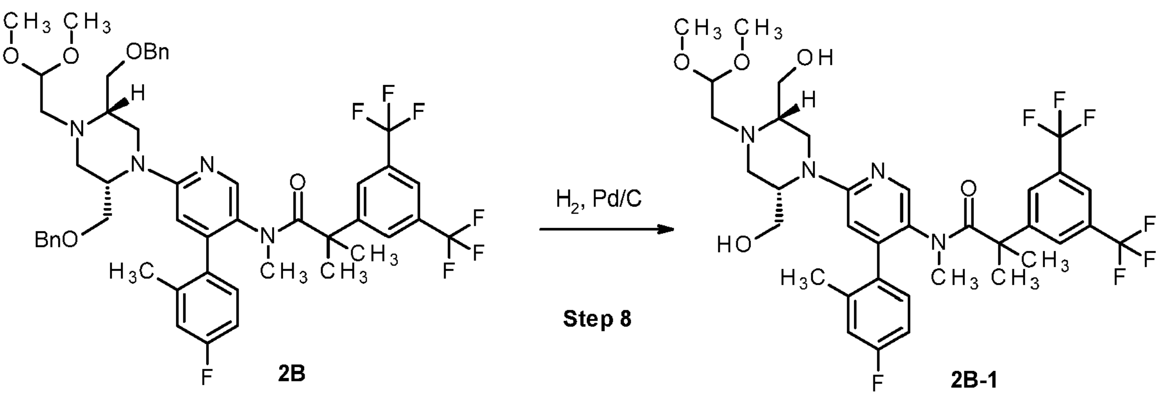


2B 2B-1

Two batches were carried out.

To a solution of intermediate 2B (60.0 g, 65.86 mmol, 1.00 eq) and HCl (25.28 g, 263.46 mmol, 24.78 mL, 38% purity, 4.00 eq) in isopropanol (1.2 L) was added Pd/C (12.00 g, 11.28 mmol, 10% purity). The mixture was stirred under $H_2$ (45 psi) at 50°C for 12 h. LCMS showed intermediate 2B was consumed. The two batches were combined for work-up. The mixture was filtered and the filtrate was concentrated under reduced pressure to obtain crude product, which

was used without purification for the next step. Intermediate 2B-1 (113 g, crude) was obtained as a gray solid.

**LCMS:** Column Xtimate C18, 3μm,2.1*30mm, RT = 2.006 min, M+H = 731.3, purity: 54%

***Step 9: Preparation of 2-[3,5-bis(trifluoromethyl)phenyl]-N-{4-(4-fluoro-2-methylphenyl)-6-[(7S,9aS)-7-(hydroxymethyl)-3-methylhexahydropyrazino[2,1-c][1,4]oxazin-8(1H)-yl]pyridin-3-yl}-N,2-dimethylpropanamide (compound of formula IV)***

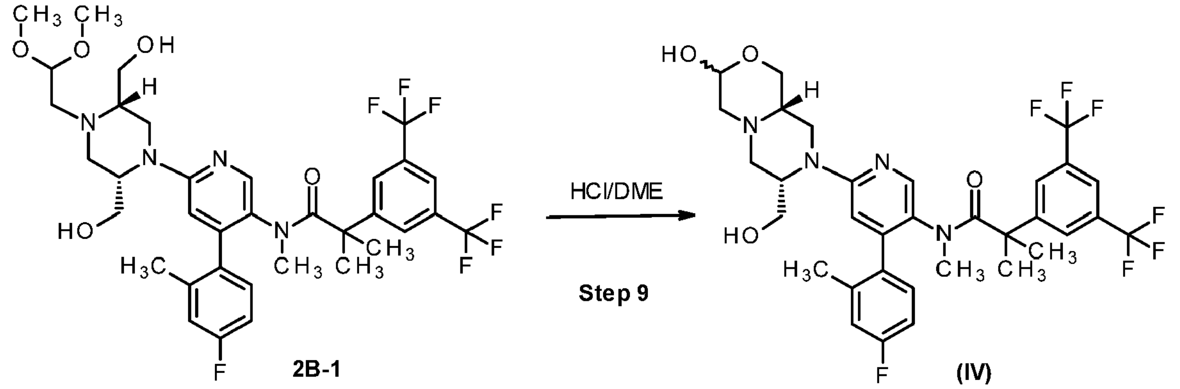


2B-1 (IV)

Intermediate 2B-1 (120 g, 164.22 mmol, 1.00 eq) in DME (1.2 L) and HCl (408.00 g, 4.25 mol, 400 mL, 38% purity, 25.89 eq) was stirred at 90°C for 12 h. LCMS showed the intermediate 2B-1 was consumed. The mixture was adjusted to pH = 7-8 with saturated $NaHCO_3$ solution, then extracted with DCM (800 mL × 2). The combined organic layers were washed with brine (500 mL), dried over $Na_2SO_4$, filtered and concentrated under reduced pressure to give a residue. The residue was first purified by flash silica gel chromatography (Ethyl acetate = 100%), then purified by preparative HPLC (column: Phenomenexluna C18 250*80mm*10 μm; mobile phase: water (10mM $NH_4HCO_3$)-ACN];B%: 45%-78%). 2-[3,5-bis(trifluoromethyl)phenyl]-N-{4-(4-fluoro-2-methylphenyl)-6-[(7S,9aS)-7-(hydroxymethyl)-3-methylhexahydropyrazino[2,1-c][1,4]oxazin-8(1H)-yl]pyridin-3-yl}-N,2-dimethylpropanamide (formula (IV))(33.0 g, 48.20 mmol, 29.35% yield) was obtained as a white solid analysed by $^1$HNMR.

**LCMS:** Column Xtimate C18, 3μm,2.1*30mmRT = 1.932 min, M+H = 685.2, purity: 50%

**$^1$H-NMR:** (400 MHz, $CDCl_3$), δ = 7.96 (s, 1H), 7.77 (s, 1H), 7.65 (s, 2H), 7.24 (s, 1H), 6.95 (s, 3H), 6.49 - 6.42 (m, 1H), 5.16 (d, J=6.5 Hz, 1H), 4.88 (s, 1H), 4.50 (s, 1H), 4.15 - 3.79 (m, 6H), 3.58 (dd, J=3.3, 11.4 Hz, 1H), 3.44 (t, J=10.9 Hz, 1H), 3.09 - 2.90 (m, 3H), 2.84 (d, J=11.1 Hz, 1H), 2.64 - 1.95 (m, 9H), 1.82 (s, 3H), 1.54 - 1.20 (m, 6H)

**Example 3: 2-[3,5-bis(trifluoromethyl)phenyl]-N-{4-[4-fluoro-2-(hydroxymethyl)phenyl]-6-[(7S,9aS)-7-(hydroxymethyl)-3-methylhexahydropyrazino[2,1-c][1,4]oxazin-8(1H)-yl]pyridin-3-yl}-N,2-dimethylpropanamide (compound of formula III) according to Scheme 3, *supra***

***Step 1: Preparation of intermediate 1***

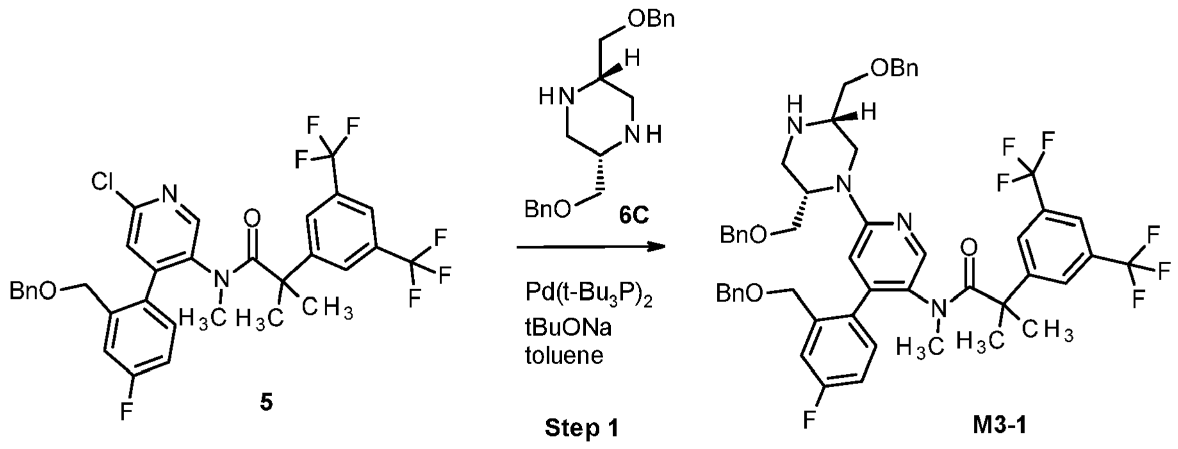


To a mixture of intermediate 6C (Example 2, step 5, above; 10.0 g, 30.6 mmol), intermediate 5 (Example 1, step 7, above; 19.6 g, 30.6 mmol) and t-BuONa (5.15 g, 53.6 mmol) in toluene (300 mL) was added Pd(t-Bu$_3$P)$_2$ (1.57 g, 3.06 mmol). The mixture was stirred at 80°C for 8 hours. LCMS showed the starting material was consumed completely. The mixture was concentrated under reduced pressure to afford crude product. The crude product was purified by column chromatography (SiO$_2$, CH$_2$Cl$_2$/MeOH=1%-10%) to obtain intermediate M3-1 (9.00 g, 9.69 mmol, 31.6% yield) as a yellow oil.

***Step 2: Preparation of intermediate 2***

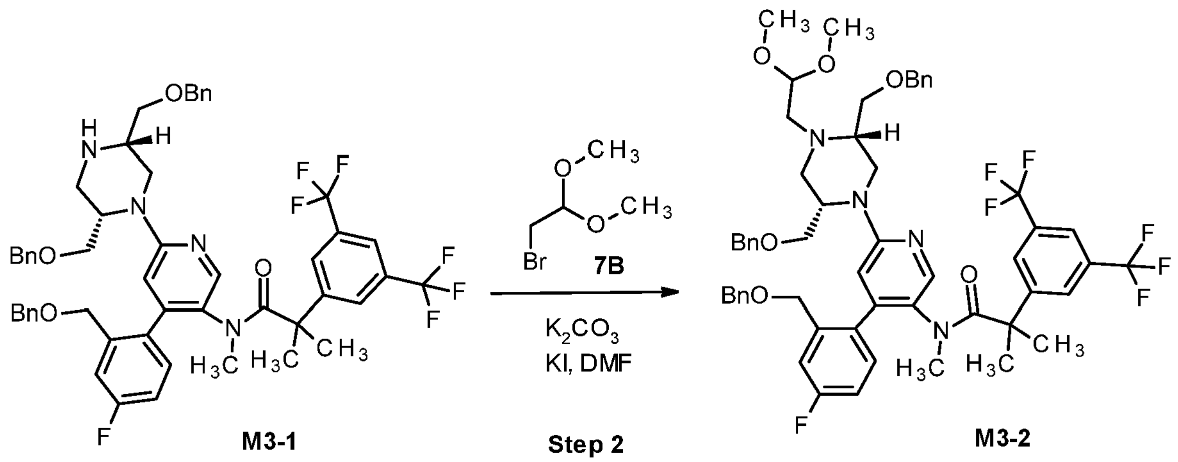


A mixture of intermediate M3-1 (9.00 g, 9.69 mmol), intermediate 7B (Example 2, step 7, above; 22.9 g, 135.6 mmol, 15.9 mL), K$_2$CO$_3$ (4.02 g, 29.1 mmol) and KI (321 mg, 1.94 mmol) in DMF (100 mL) was stirred at 100 °C for 24 hours. LCMS showed the starting material was consumed completely and the desired MS was detected. The mixture was poured into water (300 mL) and extracted with EtOAc (200 mL × 3). The combined organic layers were washed with brine (200 mL), dried over anhydrous Na$_2$SO$_4$ and concentrated under reduced pressure. The crude product was purified by silica gel column chromatography (Petroleum ether: Ethyl acetate = 2 : 1) to afford intermediate M3-2 (6.90 g, 6.51 mmol, 67.2% yield, 96.0% purity) as a yellow oil analysed by $^1$H-NMR.

**LCMS:** Column Xtimate C18,3um,2.1*3mm; RT = 2.88 min, M+H = 1017.4, purity = 96.4%. **$^1$H-NMR:** (CDCl$_3$, 400 MHz) δ 7.90 - 8.05 (m, 1H), 7.75 - 7.88 (m, 1H), 7.60 - 7.75 (m, 2H), 7.38 - 6.87 (m, 21H), 6.52 - 6.46 (m, 1H), 4.51 - 4.20 (m, 10H), 3.95 - 3.75 (m, 1H), 3.65 - 3.55 (m, 2H), 3.45 (s, 1H), 3.33 (s, 6H), 3.20 - 3.30 (m, 1H), 3.06 - 3.02 (m, 1H), 2.95 - 2.75 (m, 1H), 2.65 - 2.46 (m, 4H), 2.23 (s, 2H), 1.57 (s, 3H), 1.52 (s, 2H), 1.36 (s, 3H), 1.19 (s, 1H).

***Step 3: Preparation of intermediate 3***

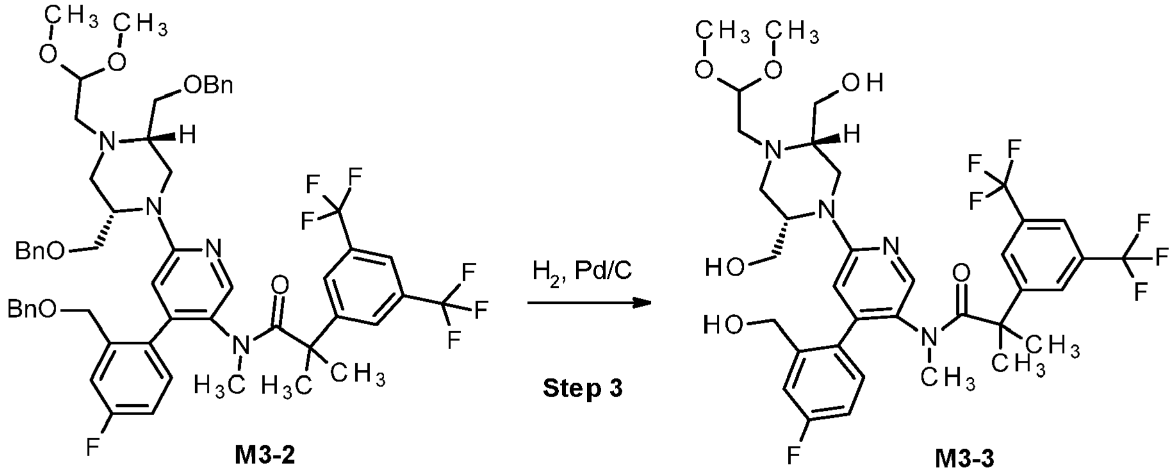


M3-2 M3-3

To a solution of intermediate M3-2 (6.00 g, 5.90 mmol) in i-PrOH (200 mL) was added HCl (2.99 g, 29.5 mmol, 2.93 mL, 36% purity) and Pd/C (1.00 g, 10% purity). The mixture was degassed and purged with $H_2$ three times. The mixture was stirred at 50 °C for 32 hours at 45 psi under $H_2$. LCMS showed the starting material was consumed completely and the desired MS was detected. The mixture was filtered through a pad of celite. The filter cake was washed with THF (50.0 mL × 3). The filtrate was concentrated under reduced pressure to obtain intermediate M3-3 (5.40 g, crude) as a pale green solid and analysed by HNMR.

**LCMS:** Column Xtimate C18, 3μm, 2.1*30mm; RT = 3.16 min, M+H = 715.4, purity = 72.2%. **$^1$H-NMR:** (DMSO, 400 MHz) δ 8.01 (s, 1H), 7.92 (s, 1H), 7.76 - 7.67 (m, 2H), 7.35 - 7.33 (m, 1H), 7.25 - 7.00 (m, 2H), 6.88 - 6.85 (m, 1H), 5.37 - 5.29 (m, 1H), 4.65 - 4.42 (m, 3H), 4.14 - 3.90 (m, 7H), 3.46 - 3.41 (m, 5H), 3.29 - 3.05 (m, 2H), 2.83 - 2.78 (m, 1H), 2.25 (s, 1H), 1.58 - 1.16 (m, 7H).

***Step 4: Preparation of 2-[3,5-bis(trifluoromethyl)phenyl]-N-{4-[4-fluoro-2-(hydroxymethyl)phenyl]-6-[(7S,9aS)-7-(hydroxymethyl)-3-methylhexahydropyrazino[2,1-c][1,4]oxazin-8(1H)-yl]pyridin-3-yl}-N,2-dimethylpropanamide (compound of formula III)***

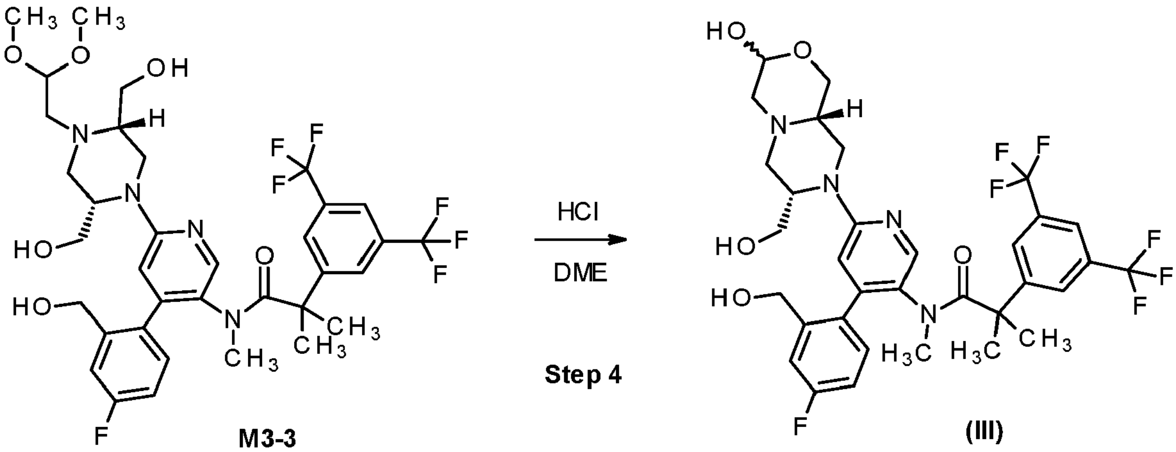


M3-3 (III)

A mixture of intermediate M3-3 (2.65 g, 3.71 mmol) in DME (21 mL) and HCl (7.14 g, 70.5 mmol, 7 mL, 36% purity) was stirred at 80 °C for 12 hours. LCMS showed 29% of the starting material was remained and 26% of the desired MS was detected. To the mixture was added water (100 mL) and adjusted pH = 7 by adding aq. saturated $NaHCO_3$ solution. The mixture was extracted with $CH_2Cl_2$ (100 mL × 3). The combined organic layers were washed with brine (50 mL), dried over anhydrous $Na_2SO_4$ and concentrated under reduced pressure. The mixture was purified by preparative HPLC (Column: Phenomenex Gemini-NX C18 75*30 mm*3 μm; Condition: water (10mM $NH_4HCO_3$)-ACN; Begin B: 35; End B: 55; Gradient Time(min): 14; 100%B Hold Time(min): 3; FlowRate (ml/min): 25) to obtain the compound of formula (III) (915 mg, 1.29 mmol, 17.4% yield, 98.9% purity) as a yellow solid analysed by $^1$H-NMR.

**LCMS:** Column Xtimate C18, 3μm, 2.1*30mm; RT = 1.81 min, M+H = 701.4, purity = 99.5%.
**$^1$H-NMR:** ($CDCl_3$, 400 MHz) δ 7.93 - 7.92 (m, 1H), 7.79 (s, 1H), 7.66 (s, 2H), 7.50 - 7.30 (m, 1H), 6.96 (s, 2H), 6.51 - 6.42 (m, 1H), 5.17 (s, 0.4H), 5.00 - 4.80 (m, 0.6H), 4.65 - 4.61 (m, 1H), 4.60 - 4.45 (m, 1H), 4.45 - 4.25 (m, 1H), 4.00 - 3.83 (m, 6H), 3.70 - 3.55 (m, 0.6H), 3.55 - 3.35 (m, 0.8H), 3.30 - 3.10 (m, 0.5H), 3.10 - 2.90 (m, 2.7H), 2.90 - 2.80 (m, 0.6H), 2.71 (s, 2.5H), 2.62 - 2.34 (m, 3H), 2.04 - 1.84 (m, 1H), 1.43 - 1.25 (m, 5H), 1.14 (s, 3H).

**Example 4: N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula II)**

**1. Small Scale Production**

0.5 g of 2-[3,5-bis(trifluoromethyl)phenyl]-N-{4-(4-fluoro-2-methylphenyl)-6-[(7S,9aS)-7-(hydroxymethyl)hexahydropyrazino[2,1-c][1,4]oxazin-8(1H)-yl]pyridin-3-yl}-N,2-dimethylpropanamide (Elinzanetant, previously disclosed in e.g. WO 2007/028654 A1, Example 34) were suspended in 50 ml of 10 % $H_2O_2$ solution.

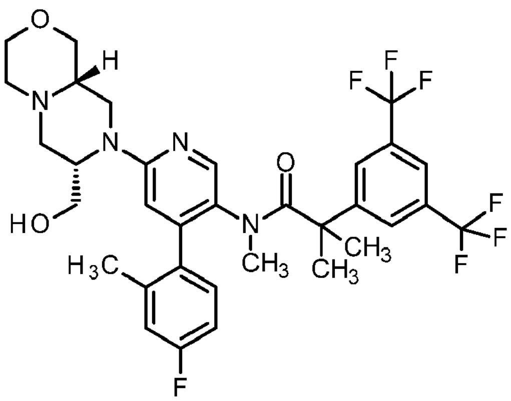

Elinzanetant N-[6-[(7S,9aS)-7-(hydroxymethyl)-3,4,6,7,9,9a-hexahydro-1H-pyrazino[2,1-c][1 ,4]oxazin-8-yl]-4-(4-fluoro-2-methyl-phenyl)-3-pyridyl]-2-[3,5-bis(trifluoromethyl)phenyl]-N,2-dimethylpropanamide

The mixture was heated up to reflux temperature, maintained for 5 hours and cooled down. The supernatant was discarded and the resin stuck in the bottom of the reactor was dissolved in 5 ml of MTBE.
The resulting MTBE solution was analyzed by HPLC and the profile showed 3 main compounds (Elinzanetant was not detected):

1: (M+1 = 657, unknown): 7 % a.d.

2: (M+1 = 530, unknown): 10.2 % a.d.

3: N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)): 46.7 %

**Batch 1**

The same procedure as for previous experiment was performed using 3 % $H_2O_2$ solution. After 7 hours at reflux temperature, the profile of the resulting MTBE solution showed:

1: N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)): 29 %

2: 2-[3,5-bis(trifluoromethyl)phenyl]-N-{4-(4-fluoro-2-methylphenyl)-6-[(7S,9aS)-7-(hydroxymethyl)hexahydropyrazino[2,1-c][1,4]oxazin-8(1H)-yl]pyridin-3-yl}-N,2-dimethylpropanamide (Elinzanetant): 62 %

The solvent was evaporated using $N_2$ flow and the residue was codified as Batch 1.

**Batch 2**

0.5 g of 2-[3,5-bis(trifluoromethyl)phenyl]-N-{4-(4-fluoro-2-methylphenyl)-6-[(7S,9aS)-7-(hydroxymethyl)hexahydropyrazino[2,1-c][1,4]oxazin-8(1H)-yl]pyridin-3-yl}-N,2-dimethylpropanamide (Elinzanetant) were suspended in 50 ml 3% $H_2O_2$ solution and the mixture was heated up to reflux temperature for 28 hours. 25 ml of dichloromethane were loaded and layers were separated after 2 hours, the resulting aqueous layer was turbid. First organic layer was distilled under vacuum to dryness and 0.35 g of N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)) (76.2 %a) were obtained.

Overnight, an organic layer appeared from aqueous layer. It was separated and analyzed by HPLC (69.3 %a). N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)) was contained.

**Batch 3**

Batch 1 and Batch 2 were combined and purified by flash chromatography employing mixtures of DCM:MeOH 95:5 and 90:10.

0.326 g of N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)) (Batch 3) were obtained.

## 2. Scale Up Production

**Batch 4**

15.00 g of 2-[3,5-bis(trifluoromethyl)phenyl]-N-{4-(4-fluoro-2-methylphenyl)-6-[(7S,9aS)-7-(hydroxymethyl)hexahydropyrazino[2,1-c][1,4]oxazin-8(1H)-yl]pyridin-3-yl}-N,2-dimethylpropanamide (Elinzanetant) were oxidized with 400 ml 3% $H_2O_2$ solution, at reflux temperature. MTBE (10 mL) was loaded initially to dissolve the starting material. Additional MTBE was added over time in order to maintain the solution (internal temperature was kept above 70°C).

After 24 hours at 70°C to 74 °C, the reaction mixture was cooled down and extracted with 150 ml DCM. 5 extra DCM washes were performed with 100 ml. All organic layers were combined and distilled to dryness.

24.06 g of a residue (Batch 4) were obtained as crude product.

**Purification of Batch 4**

Batch 4 was purified byflash chromatography employing mixtures of DCM:MeOH 95:5 and 90:10. Fractions were combined and distilled to dryness.

11.41 g of N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)) as yellowish solid were obtained.

## 3. Spectroscopic Characterisation of Batch 4

### 3.1 IR Spectroscopy

The spectrum as observed by IR Spectroscopy is shown in Figure 2.

### 3.2 NMR Spectroscopy

#### 3.2.1 $^1$H-NMR Conditions and Results

The proposed structure for N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide (compound of formula (II)) is shown below. In addition, the atoms have been numbered for an easier identification.

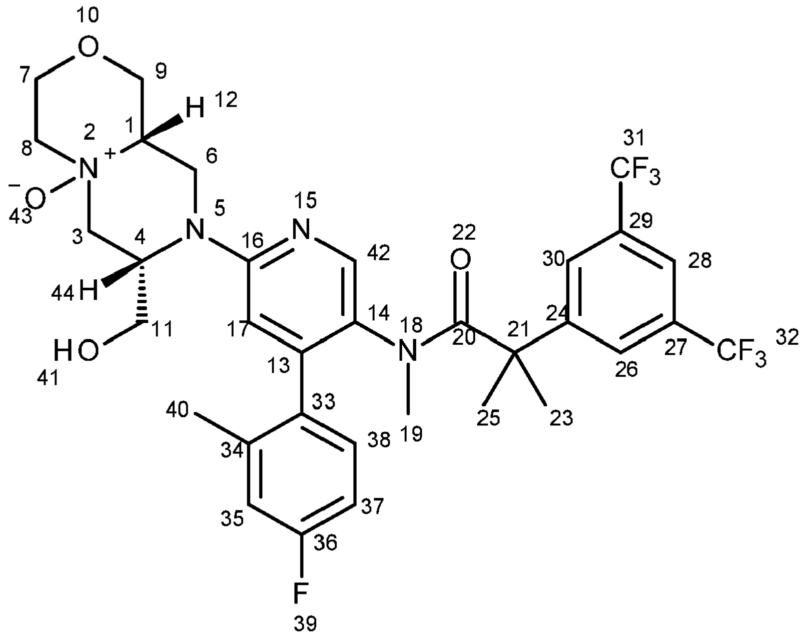

formula (II)

$C_{33}H_{35}F_7N_4O_4$

Exact Mass: 684.2547

The $^1$H-NMR spectra of this example were recorded on an Agilent (Varian Mercury 400) spectrometer, using the standard acquisition conditions: spectrometer frequency: 400.13 MHz; number of scans: 16; spectral width: 6400 Hz (from -2 to 14 ppm); pulse width: 4.7 µs; interpulse delay: 5 s; solvent: DMSO-d6; reference: residual semi-deuterated solvent (δ 2.50).

3.2.1.1 Spectrum at 25 °C

Figure 3 shows the spectrum at 25°C, some of the proton signals of the molecule appeared to be extremely broad due to the co-existence of conformational equilibria at room temperature. In particular, protons of the methyl groups 23 and 25 resonated as a triple broad signal between 1.58 and 1.11 ppm. The same effect was observed on the aromatic methyl group 40 and on the N-methyl group 19, which apparently resonated as broad signals between 2.23 and 2.04 ppm.

The signals from the $CH_2$/CH protons of the bicycle appeared between 4.70 and 3.05 ppm, and the peaks corresponding to the aromatic protons between 8.06 and 6.64 ppm.

3.2.1.2 Spectrum at 70 °C

In order to improve peaks shape, a spectrum was performed at 70°C (see Figure 4). Under this condition, the methyl signals were clearly observed at 1.38 ppm (Me 23/25), 2.16 ppm (Me 40) and 2.45 ppm (Me 19).

The determination of the aliphatic CH and $CH_2$ signals was based on a $^1$H-$^{13}$C-HSQC spectrum (see Figure 5). According to this spectrum, the signals at 4.64 ppm and ca. 3.6 ppm have been attributed to the CH protons of the bicycle. The rest of multiplets between 4.34 ppm and 3.00 ppm corresponded to the bicycle CH2 protons.

Additionally, in order to achieve a more accurate assignment, an NOE experiment was performed by selective irradiation of the CH proton at 4.64 ppm. As can be observed in Figure 6, a clear NOE enhancement occurred on the aromatic proton H17, as well as additional NOEs on other bicyclic protons close to the irradiated proton. The fact that all the observed NOEs were negative indicated slow motion of the involved chemical groups at room temperature. A second experiment performed at 45 °C yielded the same NOE enhancements but in positive phase (see Figure 7) due to the reduction of the solvent viscosity and the increment of the molecular rotation. Thus, according to these NOE results, it was concluded that H44 can be assigned to the broad signal at 4.64 ppm and, correspondingly, the second CH of the bicycle (H12) should be assigned to a signal at approx. 3.6 ppm.

3.2.1.3. Determination of the oxidation position

The presence of three signals from methyl groups in the spectrum discarded the possibility of oxidation of the aromatic methyl. Thus, it was considered that the *N*-oxide was formed on one of the two nitrogen atoms of the bicycle.

Taking into account that H12 showed a greater chemical shift difference than H44 with respect to the chemical shift in the non-oxidized Elinzanetant, the chemical shift of H12 changes from 2.1 ppm in elinzanetant to 3.6 ppm in the *N*-oxide ($\Delta\delta$ = 1.5 ppm), while the corresponding change in the case of H44 was only from 4.24 ppm to 4.64 ppm ($\Delta\delta$ = 0.4 ppm). Hence, oxidation has taken place on nitrogen number 2 than on nitrogen number 5.

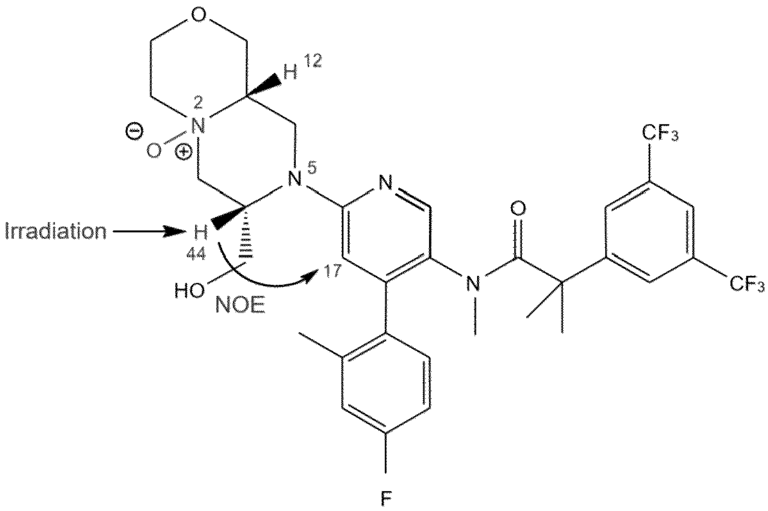


3.3 MASS SPECTROMETRY

3.3.1 Full Scan Spectrometry

The spectrum was performed on an Ion Trap Agilent, using Electrospray in the positive mode of ionization. The mobile phase was $H_2O$ 0.1 % formic acid / acetonitrile (1:1), at 1 mL/min.

As shown in Figure 8, the pseudomolecular peak appeared at *m*/*z* 685, corresponding to the [M+H] ion of the *N*-oxide.

3.3.2 MS/MS fragmentation spectrum

The ion *m*/*z* 685 was selectively fragmented (see Figure 9). Clearly, the first two fragments (m/z667 and *m*/*z* 649), although showed low intensity, can be associated with loss of $H_2O$, thus showing that the N-oxide must be adjacent to aliphatic CH/$CH_2$ groups that permitted the elimination of an $H_2O$ molecule. This fact shows again that oxidation has taken place on oneof the bicyclic nitrogen atoms, and not on the pyridine ring.

MS fragments (685): 667, 649, 637, 611, 593, 528

According to the MS results, the N-oxidation occurred on one of the nitrogen atoms of the bicyclic ring. The NMR data showed that the oxidized atom was nitrogen number 2.

**Example 5: Pharmacological analysis of the compounds according to the invention**

The purpose of this study was to test 4 compounds in Binding and cellular receptor functional assays.

1. Study Design

4 compounds as synthesized according to the above's examples, i.e. compounds of formula (II); (III), (IV), and (V), were tested at several concentrations for $IC_{50}$ or $EC_{50}$ determination .

2. Measurements

Compound binding was calculated as a %-inhibition of the binding of a radioactively labeled ligand specific for

each target.

Cellular antagonist effect was calculated as a %-inhibition of reference agonist response for each target.

3. Materials and Methods

3.1 Test Compounds

The test compounds were synthesized as disclosed herein and provided with the following specifications in the testing

| Client Compound ID | Purity | Received Form | Stock solution |
|---|---|---|---|
| Compound of formula (III) | 95.6 | Powder | $1^{-02}$ M in DMSO |
| Compound of formula (II) | 99.34 | Powder | $1^{-02}$ M in DMSO |
| Compound of formula (V) | 97.7 | Powder | $1^{-02}$ M in DMSO |
| Compound of formula (IV) | 97.29 | Powder | $1^{-02}$ M in DMSO |

*3.2 In Vitro* Pharmacology: Receptor Binding Assays

**Measurement of NK1 receptor binding affinity in receptor binding assay:**

NK1 receptor binding affinities of the compounds of the invention were determined by scintillation counting and using membrane preparations of the human astrocytoma cell line U373 MG (as disclosed in Heuillet, E. et al. (1993), J. Neurochem., 60: 868-876), that is endogenously expressing human NK1 receptors. [$^{125}$I]-SubstanceP (SP) LYS3 was used as agonistic radioligand in the binding assay at concentrations of 0.05 nM, while [$Sar^9$,$Met(O_2)$ $^{11}$]-SP (at concentrations of 1μM) served as a control to determine nonspecific binding in the assay, which is outlined in the table below.

**Measurement of NK3 receptor binding affinity in receptor binding assays:**

NK3 receptor binding affinities of the compounds of the invention were determined by an scintillation counting and using membrane preparations of CHO cells recombinantly expressing human NK3 receptors (as described by Sarau, H.M. et al. (1997), J. Pharmacol. Exp. Ther., 281: 1303-1311).

The radiolabeld NK3 receptor antagonist Osanetant ([$^3$H]SR 142801) was used at concentrations of 0,4 nM in the binding assay as an antagonistic ligand, while the NK3 receptor antagonist SB 222200 at concentrations of 10μM was used to determine nonspecific binding, which is outlined in the table below, outlining the particulars of the assays.

Receptor binding affinities towards human NK1 receptors and human NK3 receptors can also be tested in accordance with the description of the Examples in WO 2007/028654 A1.

| Assay | Source | Ligand | Cone. | Kd | Non Specific | Incubation | Detection Method |
|---|---|---|---|---|---|---|---|
| **Receptors** | | | | | | | |
| **$NK_1$ *(h)* (agonist radioligand)** | human endogenous (U373MG cells) | [$^{125}$I]-Substance P LYS3 | 0.05 nM | 0.04 nM | [$Sar^9$,Met $(O_2)$ $^{11}$]-SP (1 μM) | 30 min RT | Scintillation counting |
| **$NK_3$ *(h)* (antagonist radioligand)** | human recombin ant (CHO cells) | [$^3$H]SR 142801 | 0.4 nM | 0.47 nM | SB 222200 (10 μM) | 120 minRT | Scintillation counting |

*3.3 In Vitro* Pharmacology: Cellular and Nuclear Receptor Functional Assays

**NK1 receptor functional assay:**

Human NK1 receptor antagonistic activity assays were performed by measuring antagonistic effects of the

compounds of the the formula on the reduction agonist induced intracellular [$Ca^{2+}$] calcium concentrations in the human astrocytoma cell line U373MG using fluorimetric detection. Human astrocytoma U373MG cells do endogenously express NK1 receptors and were used in the assay according to Eistetter, H.R. et al. (1992), Glia, 6: 89-95 and [$Sar^9$,Met($O_2$)$^{11}$]-Substance P was used as agonistic stimulus of the NK1 receptor in the cells and assay. Relevant particulars of the assay are outlined in the table below.

**NK3 receptor functional assay:**

Human NK3 receptor antagonistic activity was determined by measuring antagonistic effects of the compounds of the formula on the reduction of agonist induced intracellular [$Ca^{2+}$] concentrations in CHO cells recombinantly expressing human NK1 receptors using an fluorimetric detection method. The assay was adapted from Medhurst, A.D. et al. (1999), Brit. J. Pharmacol., 128: 627-636. As an agonist to induce intracellular [$Ca^{2+}$] [$MePhe^7$]-NKB was used as outlined in the overview table below.

Functional antagonism of compounds towards human NK1 receptors and human NK3 receptors can also be tested in accordance with the description of the Examples in WO 2007/028654 A1.

| Assay | Source | Stimulus | Incubation | Measured Component | Detection Method |
|---|---|---|---|---|---|
| **Receptors** | | | | | |
| **NK1 (h) (antagonist effect)** | human endogenous (U373 cells) | [$Sar^9$,Met ($O_2$)$^{11}$]-SP (1 nM) | RT | intracellular [$Ca^{2+}$] | Fluorimetry |
| **NK3 (h) (antagonist effect)** | human recombinant (CHO cells) | [$MePhe^7$]-NKB (1 nM) | RT | intracellular [$Ca^{2+}$] | Fluorimetry |

4. Analysis and expression of results

*4.1 In Vitro* Pharmacology: Binding Assays

The results are expressed as a percent of control specific binding

$$\frac{\text{measured specific binding}}{\text{control specific binding}} * 100$$

and as a percent inhibition of control specific binding

$$100-\left(\frac{\text{measured specific binding}}{\text{control specific binding}} * 100\right)$$

obtained in the presence of the test compounds.

The $IC_{50}$ values (concentration causing a half-maximal inhibition of control specific binding) and Hill coefficients (nH) were determined by non-linear regression analysis of the competition curves generated with mean replicate values using Hill equation curve fitting

$$Y=D+\left[\frac{A-D}{1+(C/C_{50})^{nH}}\right]$$

where Y = specific binding, A = left asymptote of the curve, D = right asymptote of the curve, C = compound concentration, $C_{50}$ =$IC_{50}$, and nH = slope factor. This analysis was performed using software developed at Cerep (Hill software) and validated by comparison with data generated by the commercial software SigmaPlot® 4.0 for Windows® (© 1997 by

SPSS Inc.).

The inhibition constants ($K_i$) were calculated using the Cheng Prusoff equation

$$K_i = \frac{IC_{50}}{(1+L/K_D)}$$

where L = concentration of radioligand in the assay, and $K_D$ = affinity of the radioligand for the receptor. A scatchard plot is used todetermine the $K_D$.

*4.2 In Vitro* Pharmacology: Cellular and Nuclear Receptor Functional Assays

The results are expressed as a percent of control agonist response or inverse agonist response

$$\frac{\text{measured response}}{\text{control response}} * 100$$

and as a percent inhibition of control agonist response

$$100-(\frac{\text{measured response}}{\text{control response}} * 100)$$

obtained in the presence of the test compounds.

The $EC_{50}$ values (concentration producing a half-maximal response) and $IC_{50}$ values (concentration causing a half-maximal inhibition of the control agonist response) were determined by non-linear regression analysis of the concentration-response curves generated with mean replicate values using Hill equation curve fitting

$$Y = D + \left[\frac{A-D}{1+(C/C_{50})^{nH}}\right]$$

where Y = response, A = left asymptote of the curve, D = right asymptote of the curve, C = compound concentration, and $C_{50}$= $EC_{50}$ or $IC_{50}$, and nH = slope factor.

This analysis was performed using software developed at Cerep (Hill software) and validated by comparison with data generated by the commercial software SigmaPlot ® 4.0 for Windows ® (© 1997 by SPSS Inc.).

For the antagonists, the apparent dissociation constants ($K_B$ ) were calculated using the modified Cheng Prusoff equation

$$K_B = \frac{IC_{50}}{1+(A/EC_{50A})}$$

where A = concentration of reference agonist in the assay, and $EC_{50A}$ = $EC_{50}$ value of the reference agonist.

5. Results

Results showing an inhibition or stimulation higher than 50% proof a significant effect of the test compounds onto receptor activity modulation. Such effects were observed here and are listed in the following tables.

**Results for compound of formula (III)**

| Assay | $IC_{50}$ | $K_i$ | $K_B$ | nH |
|---|---|---|---|---|
| $NK_1$*(h)* (agonist radioligand) | $5.5^{-10}$ M | $2.4^{-10}$ M | | 1 |
| $NK_1$*(h)* (antagonist effect) | $1.1^{-09}$ M | | $1.6^{-10}$ M | |
| $NK_3$*(h)* (antagonist effect) | $2.6^{-08}$ M | | $2.3^{-09}$ M | |
| $NK_3$*(h)* (antagonist radioligand) | $2.3^{-08}$ M | $1.3^{-08}$ M | | 1 |

**Results for compound of formula (II)**

| Assay | $IC_{50}$ | $K_i$ | $K_B$ | nH |
|---|---|---|---|---|
| NK1*(h)* (agonist radioligand) | $2.4^{-09}$ M | $1.1^{-09}$ M | | 1.8 |
| NK1*(h)* (antagonist effect) | $9.2^{-10}$ M | | $1.3^{-10}$ M | |
| NK3*(h)* (antagonist effect) | $1.9^{-08}$ M | | $1.7^{-09}$ M | |
| NK3*(h)* (antagonist radioligand) | $7.7^{-09}$ M | $4.2^{-09}$ M | | 0.8 |

**Results for compound of formula (V)**

| Assay | $IC_{50}$ | $K_i$ | $K_B$ | nH |
|---|---|---|---|---|
| $NK_1$*(h)* (agonist radioligand) | $2.6^{-09}$ M | $1.1^{-09}$ M | | 1.4 |
| $NK_1$*(h)* (antagonist effect) | $8.8^{-10}$ M | | $1.3^{-10}$ M | |
| $NK_3$*(h)* (antagonist effect) | $3.3^{-08}$ M | | $3.0^{-09}$ M | |
| $NK_3$*(h)* (antagonist radioligand) | $2.0^{-08}$ M | $1.1^{-08}$ M | | 1 |

**Results for compound of formula (IV)**

| Assay | $IC_{50}$ | $K_i$ | $K_B$ | nH |
|---|---|---|---|---|
| $NK_1$*(h)* (agonist radioligand) | $3.9^{-09}$ M | $1.7^{-09}$ M | | 1.2 |
| $NK_1$*(h)* (antagonist effect) | $2.6^{-09}$ M | | $3.8^{-10}$ M | |
| $NK_3$*(h)* (antagonist effect) | $1.2^{-07}$ M | | $1.1^{-08}$ M | |
| $NK_3$*(h)* (antagonist radioligand) | $9.3^{-09}$ M | $5.0^{-09}$ M | | 1.3 |

Conclusion *in vitro* Pharmacology: compounds of formula III, II, V, IV show significant binding affinities and significant antagonistic activities towards human NK1 and NK3 receptors and are suited compounds for treatment of

NK1R and/or NK3R related diseases.

The compounds according to the invention show superior *in vitro* functional antagonistic activities at NK1 receptors and NK3 receptors.

## Claims

1. A compound of general formula (I):

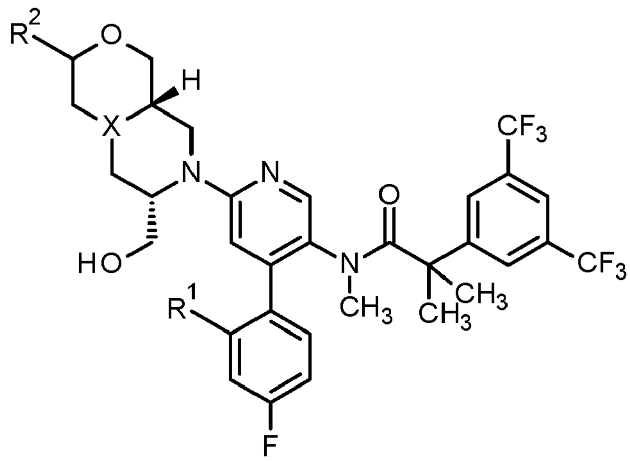

(I);

wherein

$R^1$ is $CH_3$ or $CH_2OH$;
$R^2$ is hydrogen or OH;
X is N or N-oxide.

2. The compound according to claim 1, wherein X is N if $R^1$ is $CH_2OH$ and/or $R^2$ is OH.

3. The compound according to claim 1 or 2, wherein

(i) $R^1$ is $CH_3$, $R^2$ is OH and X is N, or
(ii) $R^1$ is $CH_2OH$, $R^2$ is hydrogen and X is N, or
(iii) $R^1$ is $CH_2OH$, $R^2$ is OH and Xis N, or
(iv) $R^1$ is $CH_3$, $R^2$ is hydrogen and X is N-oxide.

4. The compound according to any one of claims 1 to 3, wherein the compound is selected from the group consisting of:

- 2-[3,5-bis(trifluoromethyl)phenyl]-N-{4-[4-fluoro-2-(hydroxymethyl)phenyl]-6-[(7S,9aS)-7-(hydroxymethyl)hexahydropyrazino[2,1-c][1,4]oxazin-8(1H)-yl]pyridin-3-yl}-N,2-dimethylpropanamide;
- 2-[3,5-bis(trifluoromethyl)phenyl]-N-{4-(4-fluoro-2-methylphenyl)-6-[(7S,9aS)-7-(hydroxymethyl)-3-methylhexahydropyrazino[2,1-c][1,4]oxazin-8(1H)-yl]pyridin-3-yl}-N,2-dimethylpropanamide;
- 2-[3,5-bis(trifluoromethyl)phenyl]-N-{4-[4-fluoro-2-(hydroxymethyl)phenyl]-6-[(7S,9aS)-7-(hydroxymethyl)-3-methylhexahydropyrazino[2,1-c][1,4]oxazin-8(1H)-yl]pyridin-3-yl}-N,2-dimethylpropanamide; and
- N-[6-[(7S,9aS)-7-(hydroxymethyl)-5-oxido-3,4,6,7,9,9a-hexahydro-1H-pyrazino[2,1-c][1,4]oxazin-5-ium-8-yl]-4-(4-fluoro-2-methyl-phenyl)-3-pyridyl]-2-[3,5-bis(trifluoromethyl)phenyl]-N,2-dimethyl-propanamide.

5. The compound according to any one of claims 1, 3, or 4, which is a compound of formula (II):

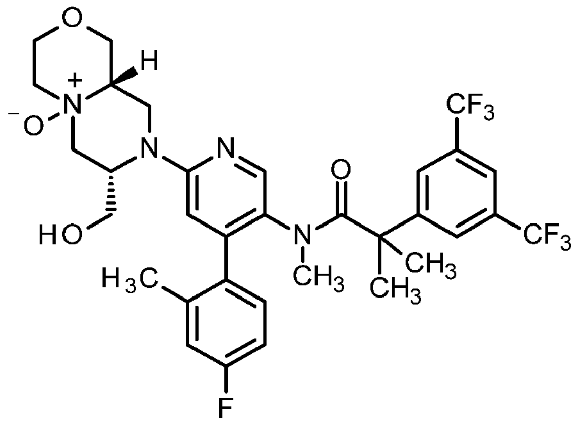

(II).

6. The compound according to any one of claims 1 to 4, which is a compound of formula (III):

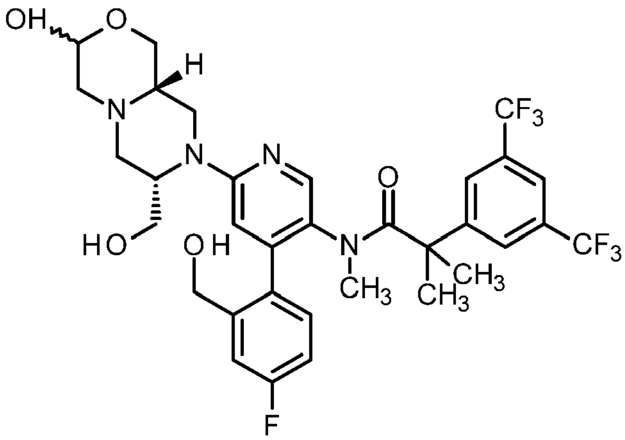

(III).

7. The compound according to any one of claims 1 to 4, which is a compound of formula (IV):

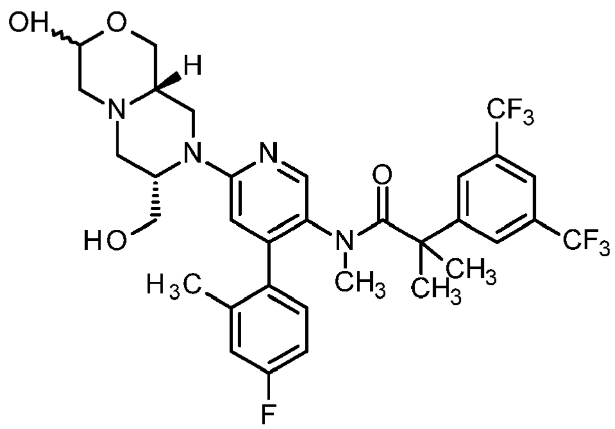

(IV).

**8.** The compound according to any one of claims 1 to 4, which is a compound of formula (V):

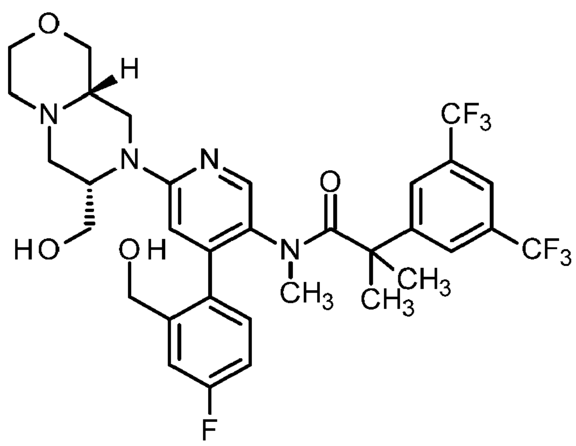

(V).

**9.** A compound of general formula (I) according to any one of claims 1 to 8 for use in the treatment or prophylaxis of a disease.

**10.** A pharmaceutical composition comprising a compound of general formula (I) according to any one of claims 1 to 8 and one or more pharmaceutically acceptable excipients.

**11.** A pharmaceutical combination comprising:

one or more first active ingredients, in particular compounds of general formula (I) according to any one of claims 1 to 8, and
one or more further active ingredients, in particular elinzanetant.

**12.** Use of a compound of general formula (I) according to any one of claims 1 to 8 for the treatment or prophylaxis of a disease.

**13.** Use of a compound of general formula (I) according to any one of claims 1 to 8 for the preparation of a medicament for the treatment or prophylaxis of a disease.

**14.** A compound for use according to claim 9, or a use according to claims 12 or 13, wherein the disease is a sex-hormone dependent disease, such as selected from the group consisting of benign prostatic hyperplasia (BPH), metastatic prostatic carcinoma, testicular cancer, breast cancer, androgen dependent acne, seborrhoea, hypertrichosis, male pattern baldness and in boys precocious puberty. Example of such diseases in women include but not limited to endometriosis, adenomyosis, abnormal puberty, uterine fibroids, heavy menstrual bleeding, hormone-dependent cancers (ovarian cancer, breast cancer), hyperandrogenism, hirsutism, hypertrichosis, female androgenetic alopecia, androgen dependent acne, seborrhoea, virilization, polycystic ovary syndrome (PCOS), HAIR-AN syndrome (hyperandrogenism, insulin resistance and acanthosis nigricans), ovarian hyperthecosis (HAIR-AN with hyperplasia of luteinized the ca cells in ovarian stroma), other manifestations of high intra ovarian androgen concentrations (e.g. follicular maturation arrest, atresia, an ovulation, dysmenorrhea, dysfunctional uterine bleeding, infertility) and androgenproducing tumor (virilizing ovarian or adrenal tumor) and osteoporosis, pre-eclampsia, hidradenitis, suppurativa and vasomotor symptoms, preferably vasomotor symptoms.

Figure 1

a)

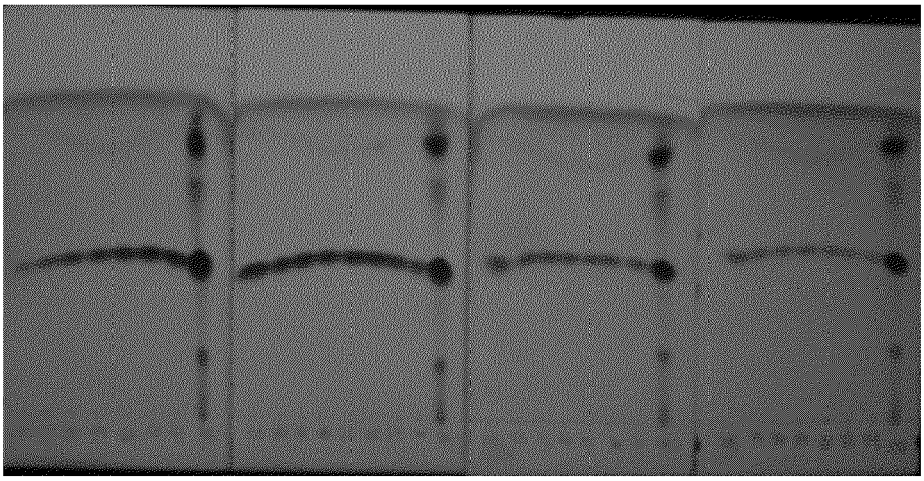

b)

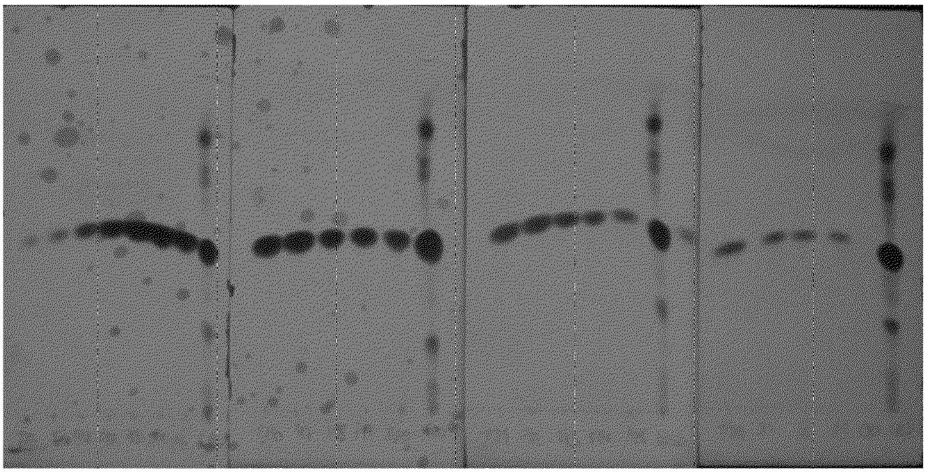

Figure 2

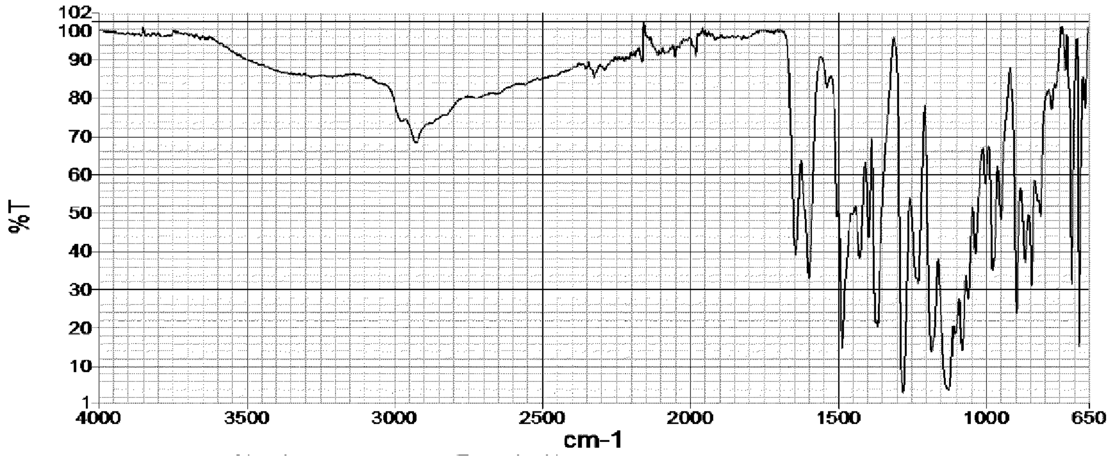
Spectrum
102
100
90
80
70
60
50
40
30
20
10
1
%T
4000
3500
3000
2500
2000
1500
1000
650
cm-1

Figure 3

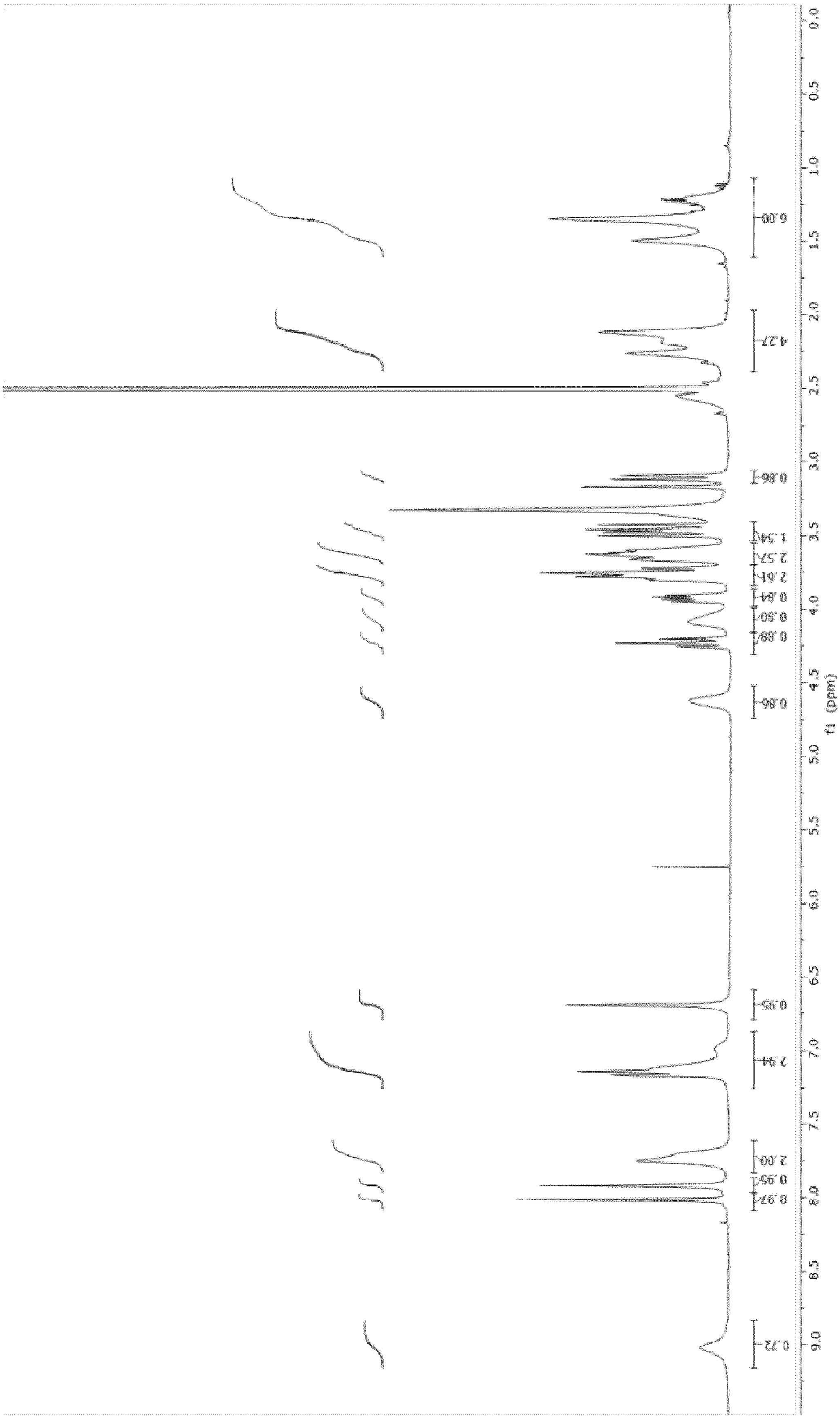
f1 (ppm)
0.0
0.5
1.0
1.5
2.0
2.5
3.0
3.5
4.0
4.5
5.0
5.5
6.0
6.5
7.0
7.5
8.0
8.5
9.0
6.00
4.27
0.86
1.54
2.57
2.61
0.84
0.80
0.88
0.86
0.95
2.94
2.00
0.95
0.97
0.72

Figure 4

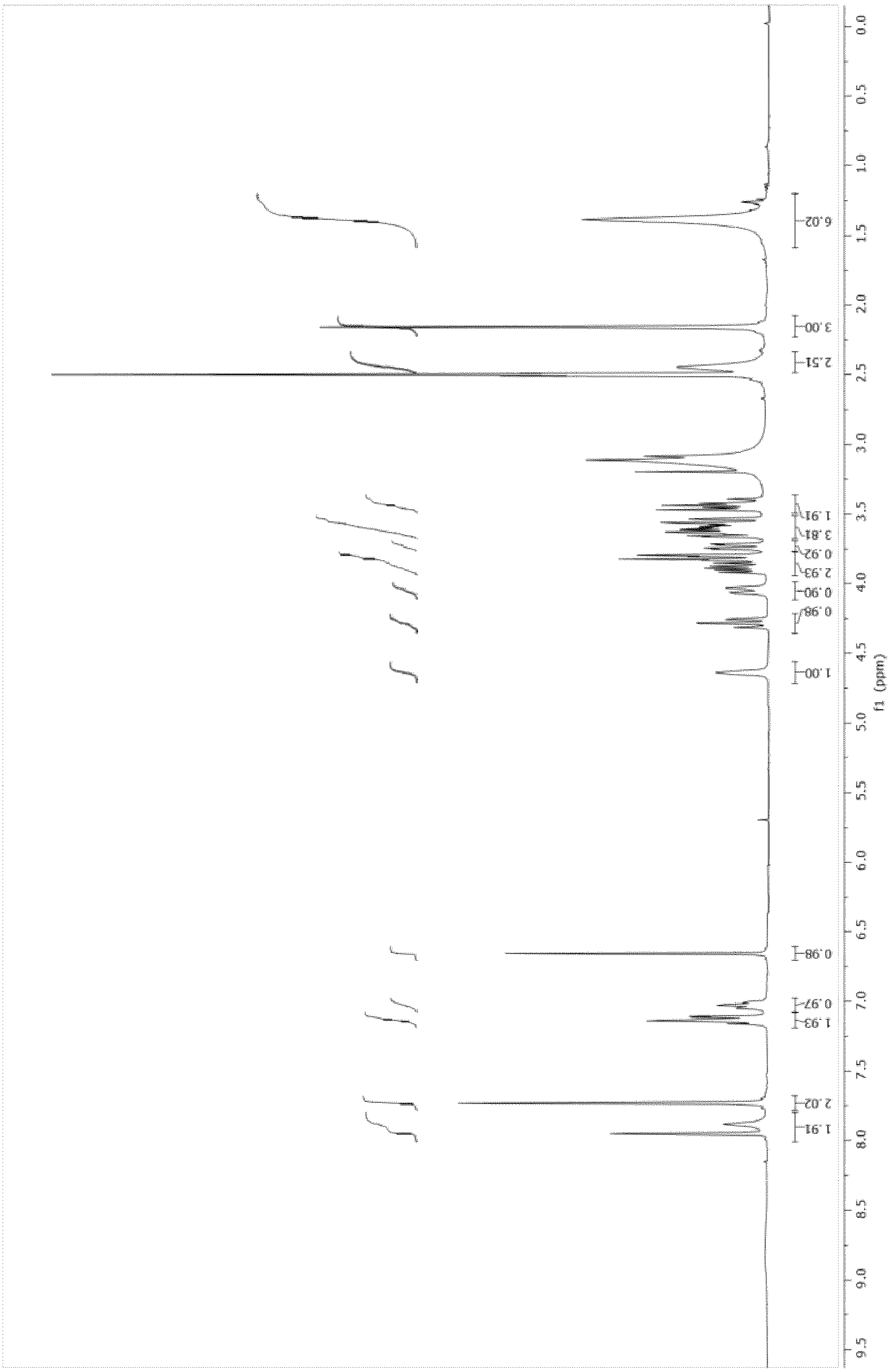
f1 (ppm)
0.0
0.5
1.0
1.5
2.0
2.5
3.0
3.5
4.0
4.5
5.0
5.5
6.0
6.5
7.0
7.5
8.0
8.5
9.0
9.5
6.02
3.00
2.51
1.91
3.81
0.92
2.93
0.90
0.98
1.00
0.98
0.97
1.93
2.02
1.91

Figure 5

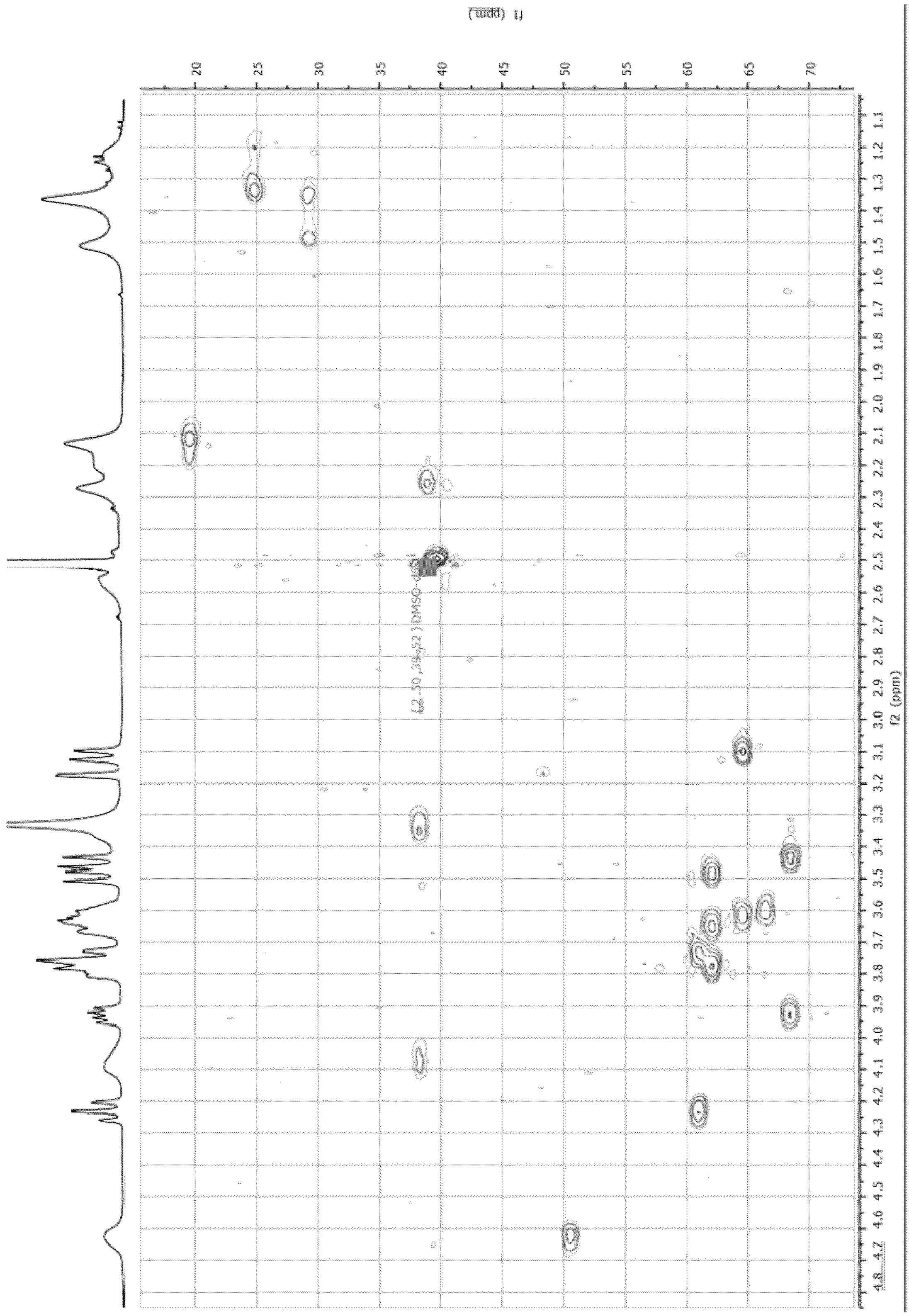

Figure 6

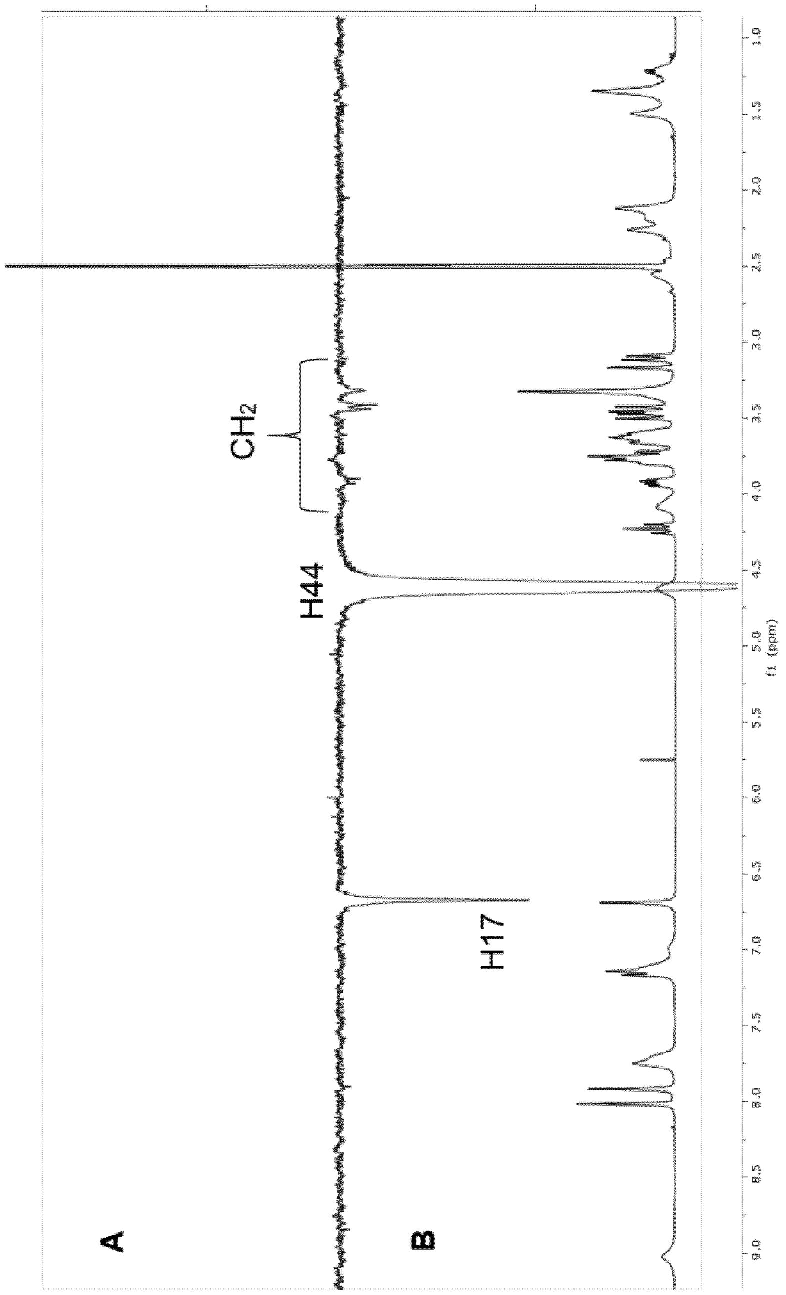
A
B
CH2
H44
H17
f1 (ppm)
9.0 8.5 8.0 7.5 7.0 6.5 6.0 5.5 5.0 4.5 4.0 3.5 3.0 2.5 2.0 1.5 1.0

Figure 7

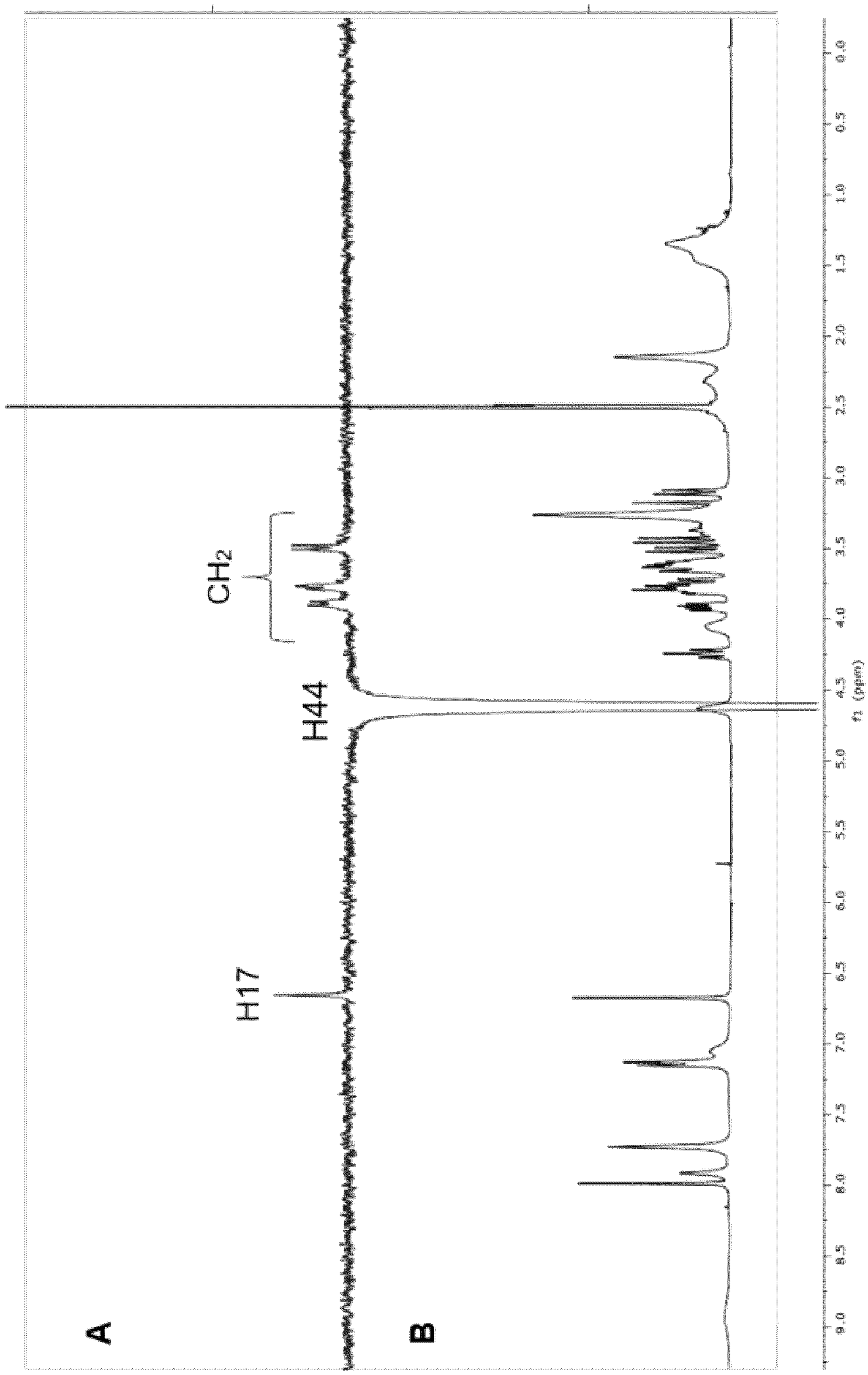
A
B
H17
H44
$CH_2$
f1 (ppm)
0.0
0.5
1.0
1.5
2.0
2.5
3.0
3.5
4.0
4.5
5.0
5.5
6.0
6.5
7.0
7.5
8.0
8.5
9.0

Figure 8

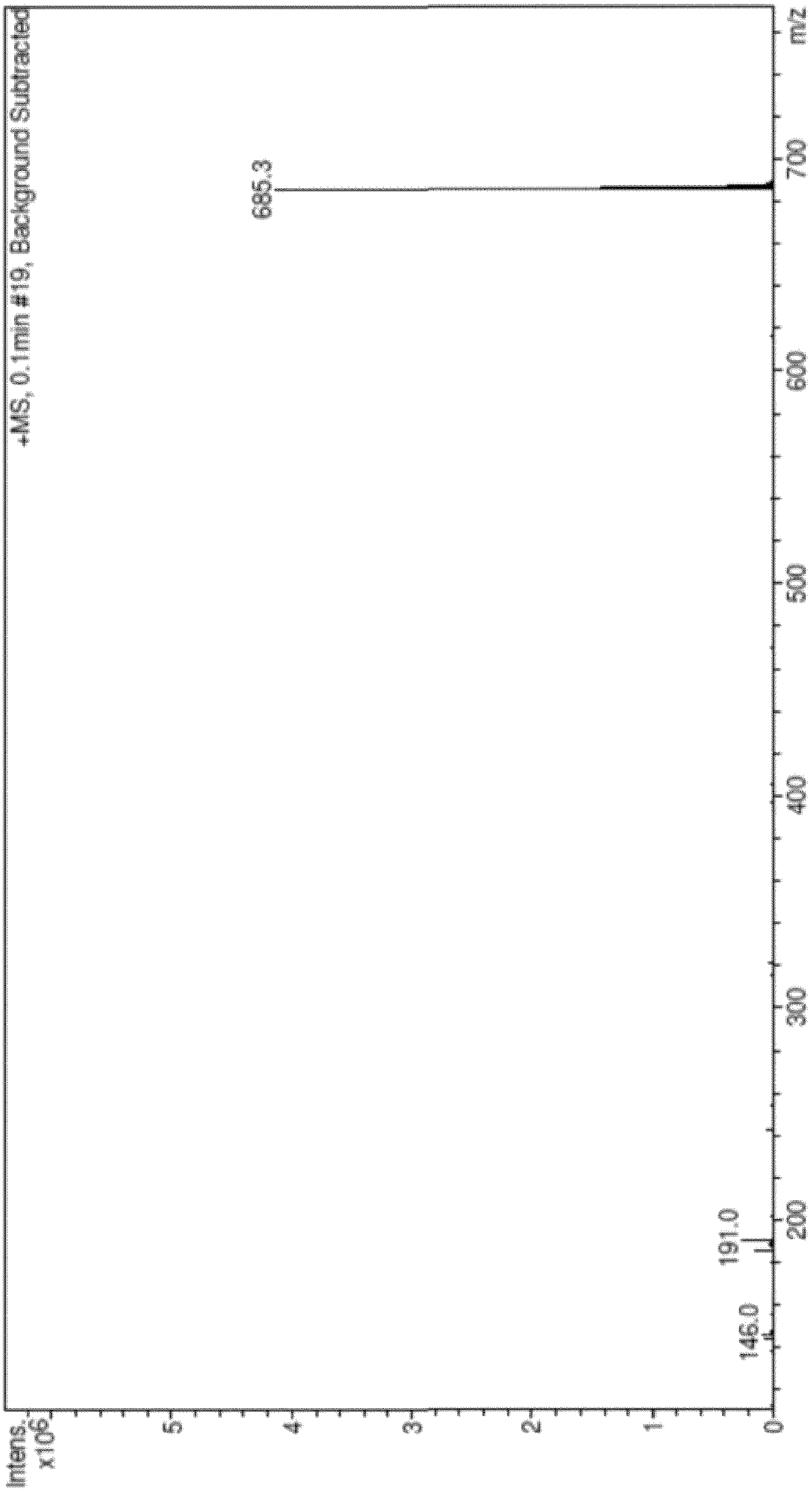
Intens.
x10^6
+MS, 0.1min #19, Background Subtracted
685.3
191.0
146.0
0
1
2
3
4
5
200
300
400
500
600
700
m/z

Figure 9

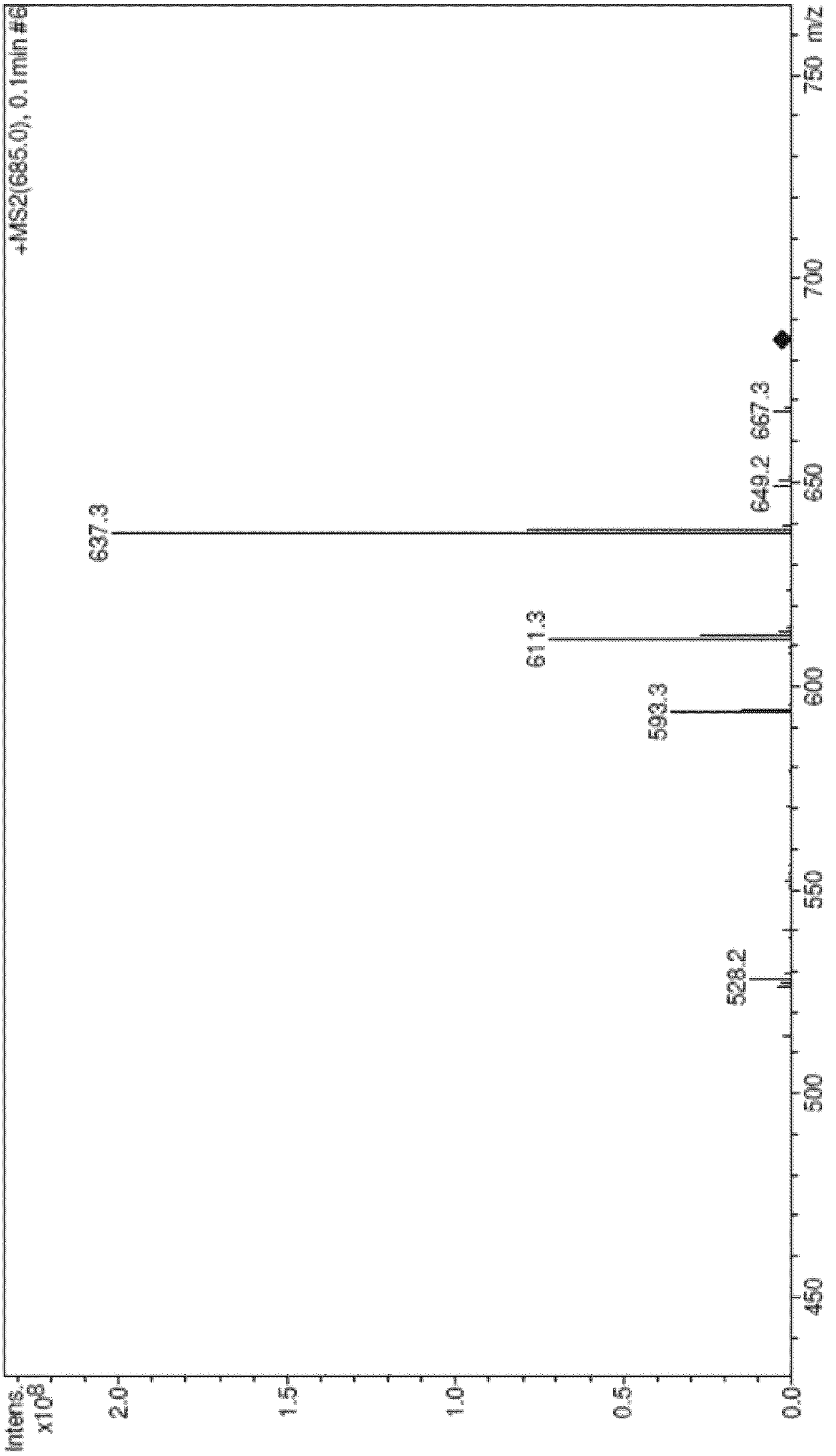

+MS2(685.0), 0.1min #6
Intens.
x10^8
2.0
1.5
1.0
0.5
0.0
637.3
611.3
593.3
528.2
649.2
667.3
450
500
550
600
650
700
750
m/z

Europäisches Patentamt
European Patent Office
Office européen des brevets

# EUROPEAN SEARCH REPORT

Application Number
EP 23 16 2389

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2007/028654 A1 (SMITHKLINE BEECHAM CORP [US]; ALVARO GIUSEPPE [IT] ET AL.) 15 March 2007 (2007-03-15) * page 185; claim 1; example 34 * ----- | 1-14 | INV. C07D498/04 A61P5/24 A61K31/5383 |
| X | WO 2016/184829 A1 (NERRE THERAPEUTICS LTD [GB]) 24 November 2016 (2016-11-24) * page 15, line 9 - line 14; claim 1; compound A * ----- | 1-14 | |

| TECHNICAL FIELDS SEARCHED (IPC) |
|---|
| C07D |

The present search report has been drawn up for all claims

1

| Place of search | Date of completion of the search | Examiner |
|---|---|---|
| Munich | 18 July 2023 | Seelmann, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 16 2389

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| WO 2007028654 A1 | 15-03-2007 | AR 058805 A1 | 27-02-2008 |
| | | AT 505472 T | 15-04-2011 |
| | | AU 2006289281 A1 | 15-03-2007 |
| | | BR PI0615787 A2 | 16-06-2009 |
| | | CA 2621564 A1 | 15-03-2007 |
| | | CR 9847 A | 29-07-2008 |
| | | CY 1111907 T1 | 04-11-2015 |
| | | DK 1928886 T3 | 11-07-2011 |
| | | EA 200800784 A1 | 30-06-2008 |
| | | EP 1928886 A1 | 11-06-2008 |
| | | EP 2336136 A1 | 22-06-2011 |
| | | HR P20110457 T1 | 31-07-2011 |
| | | IL 189578 A | 29-10-2015 |
| | | JO 2722 B1 | 15-09-2013 |
| | | JP 5121716 B2 | 16-01-2013 |
| | | JP 2009507801 A | 26-02-2009 |
| | | KR 20080046232 A | 26-05-2008 |
| | | MA 29780 B1 | 01-09-2008 |
| | | MY 145713 A | 30-03-2012 |
| | | NO 340921 B1 | 17-07-2017 |
| | | NZ 565983 A | 29-04-2011 |
| | | PE 20070614 A1 | 02-08-2007 |
| | | PL 1928886 T3 | 30-09-2011 |
| | | PT 1928886 E | 14-07-2011 |
| | | SI 1928886 T1 | 31-08-2011 |
| | | TW 200804401 A | 16-01-2008 |
| | | US 2008269208 A1 | 30-10-2008 |
| | | US 2010152175 A1 | 17-06-2010 |
| | | US 2011190276 A1 | 04-08-2011 |
| | | WO 2007028654 A1 | 15-03-2007 |
| WO 2016184829 A1 | 24-11-2016 | AU 2016264034 A1 | 26-10-2017 |
| | | AU 2020201074 A1 | 05-03-2020 |
| | | BR 112017024852 A2 | 07-08-2018 |
| | | CA 2984591 A1 | 24-11-2016 |
| | | CN 107635557 A | 26-01-2018 |
| | | CN 114272242 A | 05-04-2022 |
| | | CN 114306335 A | 12-04-2022 |
| | | CN 114306347 A | 12-04-2022 |
| | | DK 3297631 T3 | 11-11-2019 |
| | | EP 3297631 A1 | 28-03-2018 |
| | | EP 3574907 A1 | 04-12-2019 |
| | | ES 2753539 T3 | 13-04-2020 |
| | | HR P20192132 T1 | 21-02-2020 |
| | | HU E047732 T2 | 28-05-2020 |
| | | JP 6902682 B2 | 14-07-2021 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 16 2389

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| | | JP 2018515445 A | 14-06-2018 |
| | | KR 20180004815 A | 12-01-2018 |
| | | LT 3297631 T | 25-10-2019 |
| | | PL 3297631 T3 | 31-03-2020 |
| | | PT 3297631 T | 10-10-2019 |
| | | RU 2017134908 A | 18-06-2019 |
| | | SI 3297631 T1 | 31-12-2019 |
| | | US 2016339037 A1 | 24-11-2016 |
| | | US 2021128574 A1 | 06-05-2021 |
| | | US 2021236506 A1 | 05-08-2021 |
| | | WO 2016184829 A1 | 24-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011023733 A1
- WO 2005002577 A1
- WO 2007028654 A1


### Non-patent literature cited in the description


- **LEHMAN MN et al.** *Endocrinology,* 2010, vol. 151 (8), 3479-89
- **RANCE NE et al.** *J Clin Endocrinol Metab.,* 1990, vol. 71 (1), 79-85
- **RANCE NE ; YOUNG WS.** *Endocrinology,* 1991, vol. 128 (5), 2239-47
- **ROMETO AM ; RANCE NE.** *J Neuroendocrinol.,* 2008, vol. 20 (12), 1376-81
- **ABEL TW et al.** *J Clin Endocrinol Metab.,* 1999, vol. 84 (6), 2111-8
- **SANDOVAL-GUZMÁN T et al.** *J Neuroendocrinol.,* 2004, vol. 16 (2), 146-53
- **ROMETO AM et al.** *J Clin Endocrinol Metab.,* 2007, vol. 92 (7), 2744-50
- **RANCE NE et al.** *Front Neuroendocrinol.,* 2013, vol. 34 (3), 211-27
- **KNOBF MT.** *Oncologist.,* 2006, vol. 11 (2), 96-11
- **IVERSEN P et al.** *Prostate Cancer and Prostatic Diseases,* 2011, vol. 14, 184-190
- **SIMON EA et al.** *Menopause,* 31 January 2023
- **PAWSEY S et al.** *JCEM,* 2021, vol. 106 (8
- **FRASER GL et al.** *Endocrinology,* 2015, vol. 156 (11
- **S. M. BERGE et al.** Pharmaceutical Salts. *J. Pharm. Sci.,* 1977, vol. 66, 1-19
- *CHEMICAL ABSTRACTS,* 400777-00-6
- *CHEMICAL ABSTRACTS,* 289686-70-0
- *CHEMICAL ABSTRACTS,* 7252-83-7
- **HEUILLET, E. et al.** *J. Neurochem.,* 1993, vol. 60, 868-876
- **SARAU, H.M. et al.** *J. Pharmacol. Exp. Ther.,* 1997, vol. 281, 1303-1311
- **EISTETTER, H.R. et al.** *Glia,* 1992, vol. 6, 89-95
- **MEDHURST, A.D. et al.** *Brit. J. Pharmacol.,* 1999, vol. 128, 627-636